(19)

**Europäisches Patentamt**
**European Patent Office**
**Office européen des brevets**

(11)    **EP 3 054 298 B1**

(12)                **EUROPEAN PATENT SPECIFICATION**

(45) Date of publication and mention
of the grant of the patent:
**20.02.2019   Bulletin 2019/08**

(51) Int Cl.:
**G01N 33/574** [(2006.01)]

(21) Application number: **14850747.8**

(86) International application number:
**PCT/JP2014/076035**

(22) Date of filing: **30.09.2014**

(87) International publication number:
**WO 2015/050107 (09.04.2015 Gazette 2015/14)**

(54) **METHOD FOR DETECTING PANCREATIC TUMOR**

VERFAHREN ZUM NACHWEIS VON PANKREASTUMOR

PROCÉDÉ DE DÉTECTION D'UNE TUMEUR PANCRÉATIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.10.2013   JP 2013206682**
**18.08.2014   JP 2014166188**

(43) Date of publication of application:
**10.08.2016   Bulletin 2016/32**

(73) Proprietors:
• **Toray Industries, Inc.**
**Tokyo 103-8666 (JP)**
• **National Cancer Center**
**Tokyo 104-0045 (JP)**

(72) Inventors:
• **SANADA, Mitsuaki**
**Kamakura-shi**
**Kanagawa 248-8555 (JP)**
• **KOBAYASHI, Michimoto**
**Kamakura-shi**
**Kanagawa 248-8555 (JP)**
• **TAKAYAMA, Aiko**
**Kamakura-shi**
**Kanagawa 248-8555 (JP)**
• **SASAJIMA, Yoshiyuki**
**Kamakura-shi**
**Kanagawa 248-8555 (JP)**
• **JUNG, Giman**
**Kamakura-shi**
**Kanagawa 248-8555 (JP)**
• **YAMADA, Tesshi**
**Tokyo 104-0045 (JP)**
• **HONDA, Kazufumi**
**Tokyo 104-0045 (JP)**

(74) Representative: **Prüfer & Partner mbB**
**Patentanwälte · Rechtsanwälte**
**Sohnckestraße 12**
**81479 München (DE)**

(56) References cited:
**JP-A- 2004 333 274     JP-A- 2008 527 351
JP-A- 2009 103 681     JP-A- 2010 175 452
JP-A- 2010 175 452     JP-A- 2010 533 855**

• **KAZUFUMI HONDA ET AL: "Altered Plasma
Apolipoprotein Modifications in Patients with
Pancreatic Cancer: Protein Characterization and
Multi-Institutional Validation", PLOS ONE, vol. 7,
no. 10, 8 October 2012 (2012-10-08), page e46908,
XP055331189, DOI:
10.1371/journal.pone.0046908**
• **MALIK G ET AL: "Serum Levels of an Isoform of
Apolipoprotein A-II as a Potential Marker for
Prostate Cancer", CLINICAL CANCER
RESEARCH, THE AMERICAN ASSOCIATION
FOR CANCER RESEARCH, US, vol. 11, no. 3, 1
February 2005 (2005-02-01), pages 1073-1085,
XP003005157, ISSN: 1078-0432**
• **XUE AIQUN ET AL: "Discovery of diagnostic
biomarkers for pancreatic cancer in
immunodepleted serum by SELDI-TOF MS.",
PANCREATOLOGY : OFFICIAL JOURNAL OF
THE INTERNATIONAL ASSOCIATION OF
PANCREATOLOGY (IAP) ... [ET AL.], vol. 12, no.
2, March 2012 (2012-03), pages 124-129,
XP055144712, ISSN: 1424-3911**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **HONDA K ET AL.: 'Altered plasma apolipoprotein modifications in patients with pancreatic cancer: protein characterization and multi- institutional validation' PLOS ONE vol. 7, no. 10, page E46908, XP055331189**

**Description**

Technical Field

The present invention relates to a method for detecting a pancreatic tumor, i.e., pancreatic cancer or benign pancreatic tumor, comprising measuring the amounts of APOA2 protein variants in a sample of a test subject using antibodies specifically binding to APOA2 proteins (anti-APOA2 antibodies), as defined in the appended claims.

Background Art

According to the 2012 statistics, the number one cause of death in Japan is cancers. Cancers are developed from normal tissues and characterized by the formation of tumor masses caused by the abnormal growth of cancer cells, the infiltration of tumor mass-forming cancer cells to adjacent tissues, and distant metastasis to diverse organs via vascular vessels or lymph ducts. The concentrations of various proteins in the body fluids, such as blood or urine, of patients are known to vary in such onset and progression of cancers. Such proteins are called tumor markers (markers for cancer detection) and expected to be applied for various diagnostic purposes including the early detection of cancers and post-treatment follow-up (e.g., Patent Literatures 1 to 3). However, the problems of the conventional tumor markers used in clinical diagnosis are that the great majority of them offers a positive rate on the order of 50 to 70% and most of the tumor markers exhibit false-negative, particularly, for early cancers. Since advanced cancers or terminal cancers characterized by infiltration to adjacent tissues or distant metastasis result in poor prognosis, early detection is important for the effective treatment of these cancers. Hence, the discovery of a tumor marker capable of detecting early cancer with excellent sensitivity has been expected.

Pancreatic tumors refer to all tumors formed in the pancreas and are classified into pancreatic cancer, which is a malignant tumor, and benign pancreatic tumor, which is a benign tumor.

The pancreatic cancer is known as an intractable cancer among many cancers. Any effective treatment method other than surgical operation has not yet been established. Therefore, early detection or prevention of its onset is particularly important. Serum tumor markers, helical CT, a magnetic resonance imaging (MRI) apparatus, endoscopic ultrasonography (EUS), or the like is used as a method for diagnosing the pancreatic cancer (Non Patent Literature 1). However, the pancreatic cancer rarely exhibits signs in an early stage and is often detected after becoming advanced cancer. Therefore, this cancer is generally difficult to treat. Thus, there has been a demand for a novel diagnosis technique capable of accurately and easily detecting the pancreatic cancer.

On the other hand, the benign pancreatic tumor is a pathological condition at a stage previous to pancreatic cancer, and its involvement in the onset of pancreatic cancer has been suggested. However, the benign pancreatic tumor offers better prognosis than that of the pancreatic cancer and is expected to lead to the prevention of pancreatic cancer onset by its excision through surgical operation. Hence, for the benign pancreatic tumor, its early detection is also considered to be important.

The APOA2 (apolipoprotein A2 or apolipoprotein A-II) protein (GenBank Accession No. NP_001634.1) is a member of the apolipoprotein family constituting plasma lipoproteins. Ten or more apolipoproteins have heretofore been known, and their main functions are, for example, the structural stabilization of the lipoproteins, the activation of enzymes involved in lipoprotein metabolism, and effects as ligands for lipoprotein receptors present on cell surface. The APOA2 protein is synthesized as a 100-amino acid precursor containing a signal peptide in a liver tissue. Its mature form present in blood consists of 77 amino acids. The mature form of the APOA2 protein is a high-density lipoprotein (HDL)-constituting apolipoprotein having a glutamine residue (Q) at its amino terminus (N terminus), a threonine residue (T) at the 76th position counted from the N terminus, and glutamine residue (Q) at the C terminus (77th position counted from the N terminus). Also, the APOA2 protein has been reported to have variants differing in mass, including APOA2-ATQ protein (full-length APOA2 protein), APOA2-AT protein (APOA2 protein lacking the C-terminal glutamine residue (Q)), and APOA2-A protein (APOA2 protein lacking the C-terminal threonine and glutamine residues (TQ)) (Non Patent Literature 2).

According to analysis based on the conformational data of the APOA2 protein (PDB ID: 1L6L) registered in the protein structure data bank (PDB; Protein Data Bank; http://www.rcsb.org/pdb/home.do), the APOA2 proteins are dimerized through the disulfide bond (SS bond) between their cysteine residues on the N-terminal side. Thus, the APOA2 protein has been found to exist in blood as dimers having various molecular weights resulting from the combinations of the 3 variants. Specifically, these dimers are known to include, for example, a dimer consisting of the full-length APOA2-ATQ proteins (APOA2-ATQ/ATQ protein dimer), a dimer of the APOA2-ATQ protein and the APOA2-AT protein (APOA2-ATQ/AT protein dimer), a dimer consisting of the APOA2-AT proteins (APOA2-AT/AT protein dimer), a dimer of the APOA2-AT protein and the APOA2-A protein (APOA2-AT/A protein dimer), and a dimer consisting of the APOA2-A proteins (APOA2-A/A protein dimer). In addition, the APOA2 protein is also known to be dimerized with another protein such as APOD protein, APOE protein, or APOA1-M protein through a disulfide bond and to exist as a monomer (Non Patent Literatures 3 and 4).

These various APOA2 protein dimers are known to exhibit quantitative variations in the blood of pancreatic cancer patients compared with normal persons. Particularly, the APOA2-ATQ/AT protein dimer has been found as a protein having a mass value of molecular weight 17253 $\pm$ 9 (m/z) as a result of mass spectrometry. It has been revealed that: this protein dimer is significantly decreased in pancreatic cancer patients compared with normal persons; and pancreatic cancer can be detected with accuracy as high as an AUC (area under the curve) value of 0.85 or higher by using the protein dimer as a pancreatic cancer marker (Patent Literatures 4 and 5 and Non Patent Literature 5).

However, 3 complicated steps including measurement with a mass spectrometer are required for achieving the discrimination accuracy of the AUC value. For example, in the first step, a blood sample for use in mass spectrometry is pretreated with 9 M urea and 2% CHAPS. However, the urea is susceptible to degradation and thus unsuitable for long-term storage. Therefore, for constant pretreatment conditions, it is necessary to prepare a solution containing urea at every measurement. In the subsequent step, the pretreated sample is captured onto the surface of a protein chip (manufactured by Ciphergen Biosystems K.K.). In this capturing step, adsorption is carried out through the use of the properties, such as charge state or hydrophobicity, of the protein chip surface. Therefore, washing conditions or reagent preparation conditions are known to have a great impact on capturing efficiency. In the final step, the captured sample is assayed with a mass spectrometer. The mass spectrometer requires skills for operation such as laser intensity adjustment and has low throughput in handling specimens. In the case where many different proteins are contained in a sample, a signal obtained from each protein interferes with the signals of the other proteins. Therefore, such signals are difficult to attribute. In addition, the mass spectrometer still faces challenges in quantitative performance and is therefore unsuitable for diagnostic purposes, which require highly accurate measurement. Hence, the problem of this approach is high barriers against its actual use.

ELISA is known as a high-throughput, inexpensive, and practical method for evaluating many samples, as compared with mass spectrometry. ELISA, which is a versatile approach, does not require any operational skill. In addition, this approach is highly specific because of employing two antibodies and permits highly reproducible quantification using standards. Therefore, ELISA allows for comprehensive and highly quantitative analysis of the APOA2 protein variants present with different molecular weights in a sample.

Citation List

Patent Literature

Patent Literature 1: JP Patent Publication (Kokai) No. 2001-289861 A (2001)
Patent Literature 2: JP Patent Publication (Kokai) No. 2002-323499 A (2002)
Patent Literature 3: JP Patent Publication (Kokai) No. 2009-034071 A (2009)
Patent Literature 4: International Publication No. WO 2006/098087
Patent Literature 5: JP Patent Publication (Kokai) No. 2010-175452 A (2010)

Non Patent Literature

Non Patent Literature 1: Clinical Practice Guidelines for Pancreatic Cancer based on evidence-based medicine, 2009, Japan Pancreas Society (Committee for revision of clinical guidelines for pancreatic cancer), Kanahara & Co., Ltd.
Non Patent Literature 2: Pankhurst G. et al., 2003, J. Lipid Res., Vol. 44, p. 349-355
Non Patent Literature 3: Blanco-Vaca F. et al., 2001, J. Lipid Res., Vol. 42, p. 1727-1739
Non Patent Literature 4: Rocco AG. et al., 2006, Biophys J., Vol. 91, p. 3043-3049
Non Patent Literature 5: Honda K. et al., 2012, PLoS One, Vol. 7, p. e46908

Summary of Invention

Technical Problem

In general, tumor markers are used in the detection of tumors. However, pancreatic tumors are known to be difficult to detect using tumor markers. Among the pancreatic tumors, particularly, early pancreatic cancer patients are difficult to distinguish from normal persons. Therefore, the early detection of pancreatic cancer using tumor markers is very difficult. Few tumor markers capable of detecting benign pancreatic tumor have heretofore been known.

An object of the present invention is to provide a method for detecting a pancreatic tumor, i.e., pancreatic cancer or benign pancreatic tumor, using variants of a pancreatic tumor marker APOA2 protein with high detection sensitivity of the pancreatic tumor and with higher convenience and higher throughput than those of the detection methods described in Patent Literatures 4 and 5 and Non Patent Literature 5, and a kit for the detection of a pancreatic tumor.

Solution to Problem

In order to attain the object, the present inventors have first aimed to develop a method for detecting pancreatic cancer by use of an ELISA assay method targeting the APOA2-ATQ/AT protein dimer. On the basis of a usually performed method, the present inventors have then attempted to detect the APOA2-ATQ/AT protein dimer using antibodies against the respective C-terminal regions of the APOA2-ATQ protein and the APOA2-AT protein (anti-APOA2 protein terminus antibodies) constituting the APOA2-ATQ/AT protein dimer. Nonetheless, it has turned out that this dimer cannot be detected with high accuracy, probably due to interfering substances present in blood or the influence of steric hindrance caused by the binding of these two antibodies to the same antigen.

Accordingly, the present inventors have conducted diligent studies and consequently established a method for separately measuring the total amount of the APOA2-ATQ protein and the total amount of the APOA2-AT protein by use of sandwich ELISA using anti-APOA2 protein terminus antibodies specifically binding to the C-terminal regions and antibodies specifically binding to regions other than the APOA2 protein C-terminal regions (anti-APOA2 protein non-terminus antibodies) in combination. Instead of measuring the amount of the APOA2-ATQ/AT protein dimer as conventionally performed, the present inventors have obtained the measurement value of the total amount of the APOA2-ATQ protein and the measurement value of the total amount of the APOA2-AT protein, and combined the results. By use of this analysis method, pancreatic cancer patients can be discriminated from normal persons with high accuracy. The present inventors have further found an unexpected effect brought about by use of this analysis method, i.e., the detection of benign pancreatic tumor, which has been unattainable using the previously reported tumor markers. The present inventors have found that pancreatic tumors including benign pancreatic tumor and early pancreatic cancer can be detected with very high sensitivity on the basis of this technique, leading to the completion of the present invention.

The present invention encompasses the following aspects:

(1) A method for detecting a pancreatic tumor by measuring the amounts of APOA2 protein variants in a body fluid sample of a test subject, the detection method comprising: (A) a first step of measuring the amount of APOA2-ATQ protein in the sample using an anti-APOA2-ATQ terminus antibody specifically binding to a C-terminal region of the APOA2-ATQ protein comprising the amino acid sequence represented by SEQ ID NO: 1, and an anti-APOA2-ATQ non-terminus antibody binding to the amino acid sequence other than the C-terminal region; (B) a second step of measuring the amount of APOA2-AT protein in the sample using an anti-APOA2-AT terminus antibody specifically binding to a C-terminal region of the APOA2-AT protein comprising the amino acid sequence represented by SEQ ID NO: 2 and an anti-APOA2-AT non-terminus antibody binding to the amino acid sequence other than the C-terminal region; and (C) a third step of inputting, to a preset discriminant, the measurement value of the amount of APOA2-ATQ protein obtained in the first step and the measurement value of the amount of APOA2-AT protein obtained in the second step, and determining the test subject to have a pancreatic tumor when the resulting discriminant value of the test subject is statistically significantly different as compared with the measurement value or the discriminant value of a normal subject, wherein the APOA2-ATQ protein is the full length APOA2 protein and the APOA2-AT protein is the APOA2 protein lacking the C-terminal glutamine residue.

(2) The detection method according to (1), wherein the C-terminal regions of the APOA2-ATQ protein and the APOA2-AT protein each consist of a sequence comprising 6 or more consecutive amino acids including the C terminus.

(3) The detection method according to (1) or (2), wherein the discriminant is any one selected from the group consisting of a logistic regression expression, an expression prepared by analysis with a support vector machine, an expression prepared by the analysis of a neural network, and an expression prepared by discriminant analysis.

(4) The detection method according to (3), wherein the logistic regression expression comprises, as a variable, the measurement value of the APOA2-ATQ protein, the measurement value of the APOA2-AT protein, and/or the product of the measurement value of the APOA2-ATQ protein and the measurement value of the APOA2-AT protein.

(5) The method according to (4), wherein the discriminant value of the test subject obtained from the logistic regression expression is 2/3 or lower of the discriminant value of a normal subject.

(6) The detection method according to any of (1) to (5), wherein the pancreatic tumor is pancreatic cancer or benign pancreatic tumor.

(7) The detection method according to (6), further comprising a fourth step of measuring the amount of a pancreatic cancer marker CA19-9 or DU-PAN-2 in a body fluid sample of the test subject determined to have a pancreatic tumor in the third step, and determining the test subject to have pancreatic cancer when the measurement value exceeds a predetermined reference value and determining the test subject to have benign pancreatic tumor when the measurement value is equal to or lower than the reference value.

(8) The detection method according to any of (1) to (7), wherein the body fluid sample is blood, plasma, or serum.

(9) The detection method according to any of (1) to (8), wherein the pancreatic cancer is early pancreatic cancer.

Advantageous Effects of Invention

According to the present invention, a pancreatic tumor can be conveniently detected with high throughput and high sensitivity by the assay of variants of a pancreatic tumor marker APOA2 protein in blood. For example, the amounts of particular APOA2 protein variants contained in a body fluid sample, such as blood, collected from a patient can be merely measured to determine whether or not the patient has a pancreatic tumor or to evaluate the possibility of having a pancreatic tumor.

Brief Description of Drawings

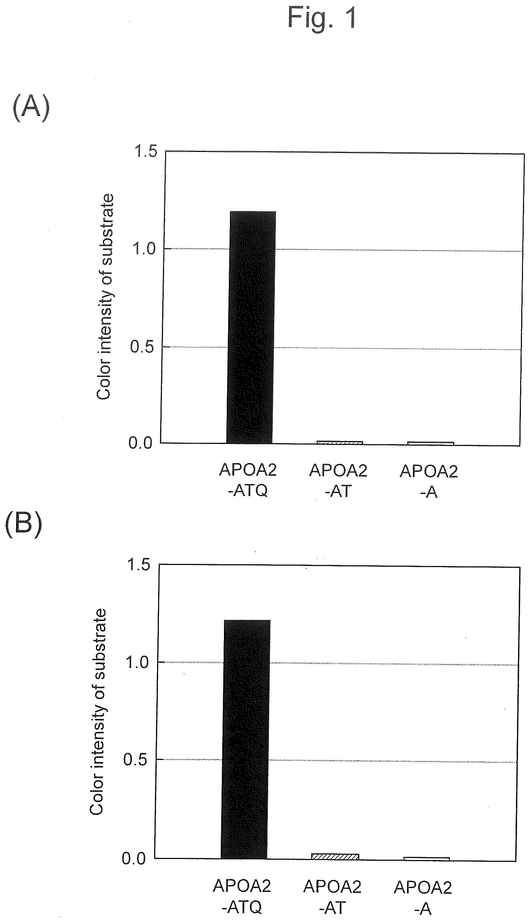

Figure 1(A) shows results of measuring the binding activity of an anti-APOA2-ATQ terminus monoclonal antibody clone 7F2 against various APOA2 protein variants. Figure 1(B) shows results of measuring the binding activity of an anti-APOA2-ATQ terminus monoclonal antibody clone 6G2 against various APOA2 protein variants.

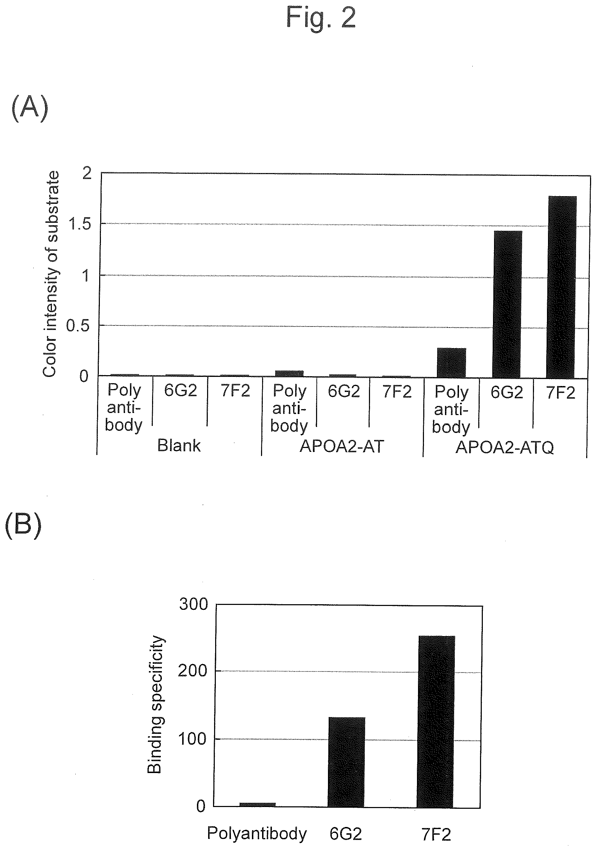

Figure 2(A) shows results of measuring the binding activity of the anti-APOA2-ATQ terminus monoclonal antibody 7F2 or 6G2 or an anti-APOA2-ATQ terminus polyclonal antibody (in the figure, indicated by "Poly antibody") against APOA2-ATQ protein or APOA2-AT protein. Figure 2(B) is a graph in which the binding specificity of each antibody was evaluated by dividing the measurement value of the binding activity of the antibody against APOA2-ATQ protein obtained in Figure 2(A) by the measurement value of its binding activity against APOA2-AT protein.

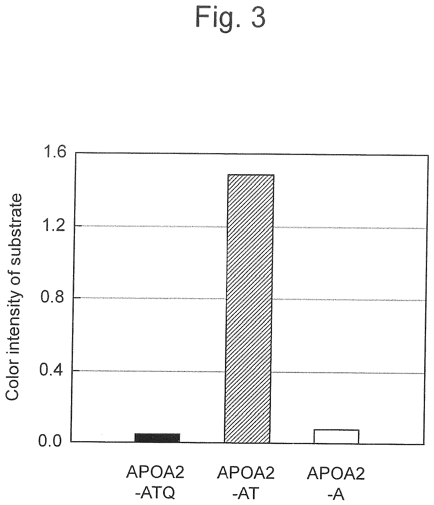

Figure 3 shows results of measuring the binding activity of an anti-APOA2-AT terminus polyclonal antibody against various APOA2 protein variants.

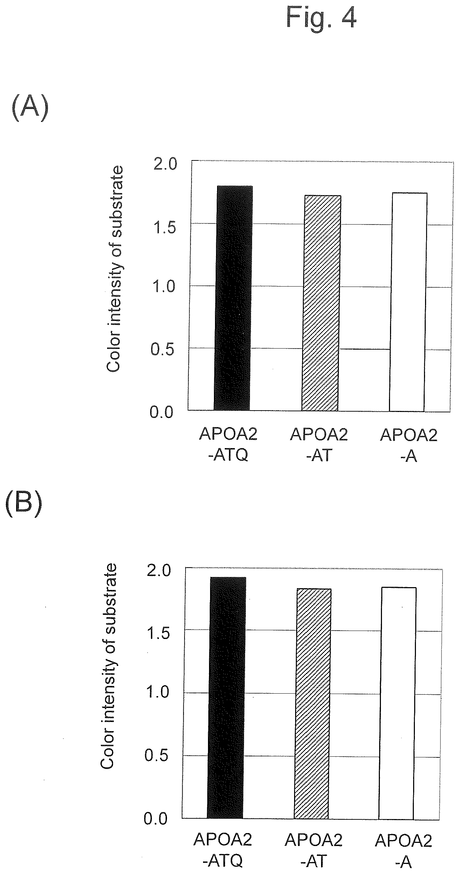

Figure 4(A) shows results of measuring the binding activity of an anti-APOA2 protein non-terminus monoclonal antibody MAB1 against various APOA2 protein variants. Figure 4(B) shows results of measuring the binding activity of an anti-APOA2 protein non-terminus monoclonal antibody MAB2 against various APOA2 protein variants.

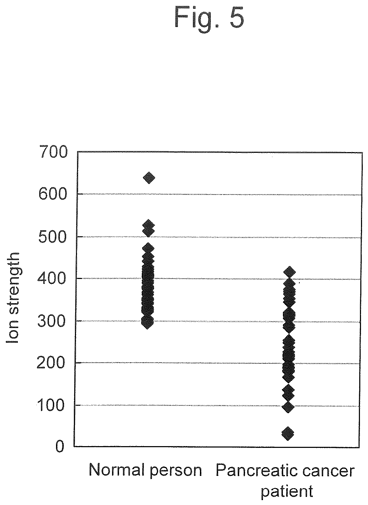

Figure 5 shows results of assaying an APOA2 protein dimer (APOA2-ATQ/AT) contained in the plasma of 40 subjects each of pancreatic cancer patients and normal persons by mass spectrometry.

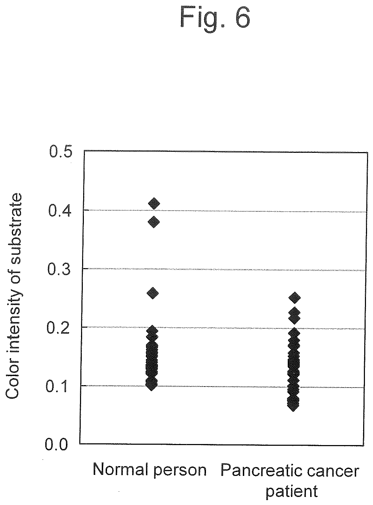

Figure 6 shows results of assaying an APOA2 protein dimer (APOA2-ATQ/AT) contained in the plasma of 40 subjects each of pancreatic cancer patients and normal persons by sandwich ELISA using a monoclonal antibody specifically recognizing the amino acid sequence of a C-terminal region of the APOA2-ATQ protein (anti-APOA2-ATQ terminus monoclonal antibody) and a polyclonal antibody specifically recognizing the amino acid sequence of a C-terminal region of the APOA2-AT (anti-APOA2-AT terminus polyclonal antibody).

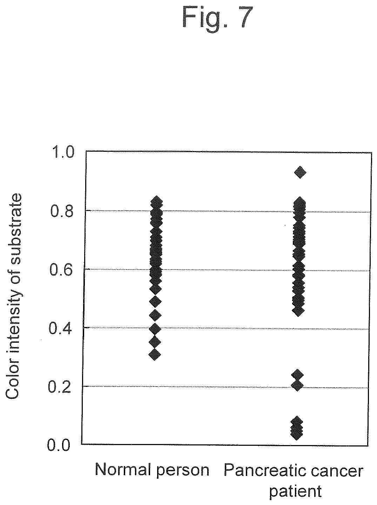

Figure 7 shows results of assaying APOA2-ATQ protein contained in the plasma of 40 subjects each of pancreatic cancer patients and normal persons by sandwich ELISA using a monoclonal antibody specifically recognizing the amino acid sequence of a C-terminal region of the APOA2-ATQ protein (anti-APOA2-ATQ terminus monoclonal antibody) and an antibody specifically recognizing an amino acid sequence other than the C-terminal region (anti-APOA2-ATQ non-terminus antibody).

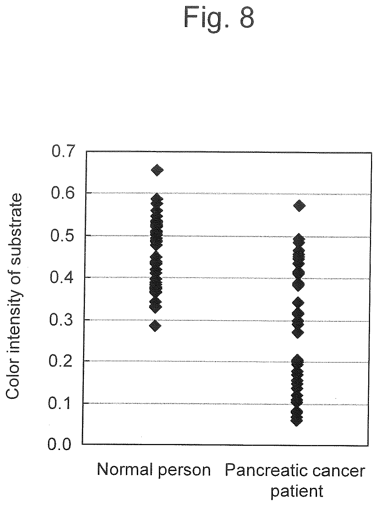

Figure 8 shows results of assaying APOA2-ATQ protein contained in the plasma of 40 subjects each of pancreatic cancer patients and normal persons by sandwich ELISA using a polyclonal antibody specifically recognizing the amino acid sequence of a C-terminal region of the APOA2-AT protein (anti-APOA2-AT terminus polyclonal antibody) and an antibody specifically recognizing an amino acid sequence other than the C-terminal region (anti-APOA2-AT non-terminus antibody).

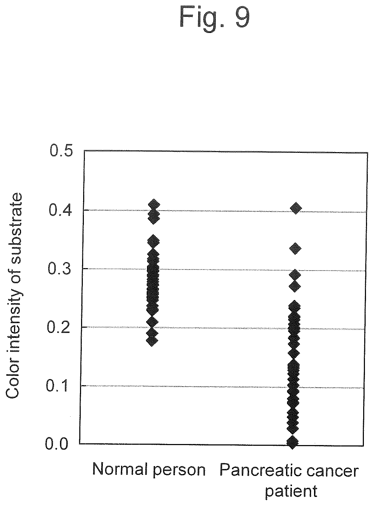

Figure 9 is a plot of the product of the amounts of two APOA2 protein variants (APOA2-ATQ protein and APOA2-AT protein) contained in the plasma of 40 subjects each of pancreatic cancer patients and normal persons.

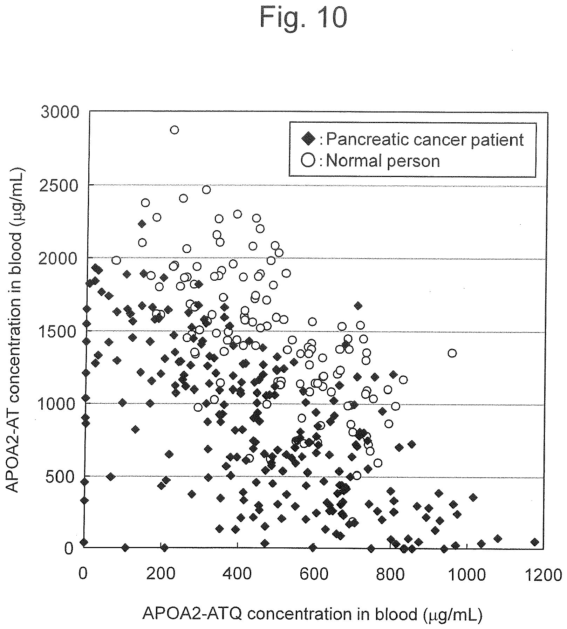

Figure 10 is a plot of the product of the concentrations of two APOA2 protein variants (APOA2-ATQ protein and APOA2-AT protein) contained in the plasma of 244 pancreatic cancer patients and 109 normal persons.

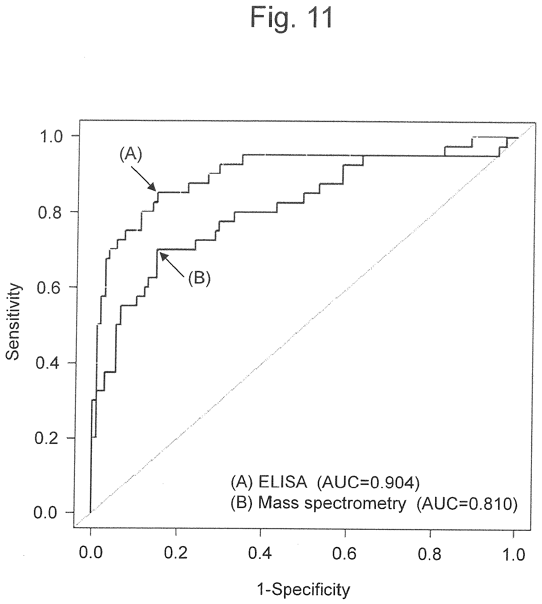

Figure 11 shows an ROC curve prepared by substituting the measurement values of pancreatic cancer patients (stages I and II in the UICC classification) and normal persons into a logistic regression expression. Figure 11(A) shows results of analyzing the product of the sandwich ELISA measurement values of two APOA2 protein variants (APOA2-ATQ protein and APOA2-AT protein). Figure 11(B) shows results of analyzing an APOA2 protein dimer (APOA2-ATQ/AT) by mass spectrometry.

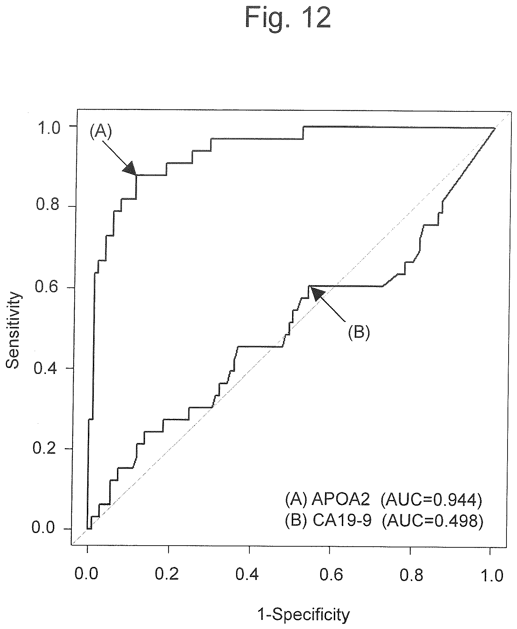

Figure 12 shows an ROC curve prepared by substituting the measurement values of benign pancreatic tumor patients and normal persons into a logistic regression expression. Figure 12(A) shows results of analyzing the product of the measurement values of two APOA2 protein variants (APOA2-ATQ protein and APOA2-AT protein) assayed by sandwich ELISA. Figure 12(B) shows results of analyzing the measurement value of CA19-9.

Description of Embodiments

The target to be assayed according to the present invention is a pancreatic tumor. In the present specification, the "pancreatic tumor" refers to every tumor formed in the pancreas. Specifically, the pancreatic tumor is "pancreatic cancer", which is a malignant tumor, or "benign pancreatic tumor", which is a benign tumor.

In the present specification, the "pancreatic cancer" refers to every malignant tumor formed in the pancreas. Specifically, the pancreatic cancer includes, for example, invasive ductal carcinoma of the pancreas, intraductal tubular carcinoma of pancreas, acinar cell carcinoma of the pancreas, serous cystadenocarcinoma of the pancreas, mucinous cystadenocarcinoma of the pancreas (one type of mucinous cystic neoplasm of the pancreas), intraductal papillary mucinous carcinoma of the pancreas (one type of intraductal papillary mucinous neoplasm of the pancreas), and neuroendocrine tumor of the pancreas (one type of endocrine neoplasm of the pancreas) ("General Rules for the Study of Pancreatic Cancer", The 6th Edition, Revised Version, 2013, Japan Pancreas Society, Kanahara & Co., Ltd.). The targeted pancreatic cancer is not limited by the degree of progression. Any of early cancer, advanced cancer, and terminal cancer is included in the scope of the present invention.

In the present specification, the "early cancer" refers to a tumor that is confined to a local area where the tumor has developed (inside of the mucous membrane) without infiltration to its neighboring tissues or with infiltration only to a limited local area. Specifically, the early cancer refers to a cancer at stage 0, IA, IB, IIA, or IIB according to the UICC (Unio Internationalis Contra Cancrum) classification ("TNM Classification of Malignant Tumours", the 7th edition, Japanese version, 2012, TNM Committee of the Japan National Committee for UICC, Kanahara & Co., Ltd.). As mentioned above, the pancreatic cancer is an intractable cancer with very poor prognosis. However, if early pancreatic cancer can be detected, 5-year survival rates can be remarkably improved.

The "benign pancreatic tumor" includes mucinous cystadenoma (one type of mucinous cystic neoplasm of the pancreas), intraductal papillary mucinous adenoma (one type of intraductal papillary mucinous neoplasm of the pancreas), neuroendocrine tumor of the pancreas (one type of endocrine neoplasm of the pancreas), serous cystadenoma, and atypical epithelium and carcinoma *in situ* occurring in the pancreas ("General Rules for the Study of Pancreatic Cancer", The 6th Edition, Revised Version, 2013, Japan Pancreas Society, Kanahara & Co., Ltd.).

1. Anti-APOA2 antibody and fragment thereof

The first aspect of the present disclosure relates to anti-APOA2 antibodies (including anti-APOA2 protein terminus antibodies and anti-APOA2 protein non-terminus antibodies) and fragments thereof.

1-1. Anti-APOA2 antibody

In the present specification, the "APOA2 protein" corresponds to an APOA2 protein of each organism species and is preferably a human-derived APOA2 protein (GenBank Accession No. NP_001634.1). Specifically, the APOA2 protein includes human-derived wild-type APOA2 protein variants shown in SEQ ID NOs: 1, 2, and 3 and further includes their natural mutants and fragments thereof.

In the present specification, the "variants" mean different molecular forms of the APOA2 protein that may be present in the plasma, serum, or other body fluids of humans or animals. The APOA2 protein variants correspond to, for example, APOA2 proteins differing in the structure of a C-terminal region, or their natural mutants. Specifically, the APOA2 protein variants correspond to, for example, APOA2-ATQ protein that is shown in SEQ ID NO: 1 and has the amino acid sequence of a C-terminal region ending in ATQ, APOA2-AT protein that is shown in SEQ ID NO: 2 and the amino acid sequence of a C-terminal region ending in AT, and APOA2-A protein that is shown in SEQ ID NO: 3 and the amino acid sequence of a C-terminal region ending in A.

In the present specification, the "C-terminal region (carboxyl-terminal region)" refers to a region consisting of 6 to 25 amino acids, preferably 8 to 20 amino acids or 10 to 17 amino acids, including an amino acid at the C terminus and a few consecutive amino acids adjacent thereto in the amino acid sequence.

In the present specification, the "natural mutant" refers to a naturally occurring mutant having, for example, an amino acid sequence derived from the amino acid sequence represented by SEQ ID NO: 1, 2, or 3 by the deletion, substitution, or addition of one or several amino acids, or having 90% or higher, 92% or higher, or 94% or higher, preferably 95% or higher, more preferably 97% or higher, further preferably 98% or higher or 99% or higher identity to the amino acid sequence. The "identity" refers to the ratio (%) of the number of identical amino acid residues in one amino acid sequence to the number of all amino acid residues (including the number of gaps) in another amino acid sequence when these two amino acid sequences are aligned with or without gaps so as to attain the largest degree of coincidence. The term "several" refers to an integer of 2 to 10, for example, an integer of 2 to 7, 2 to 5, 2 to 4, or 2 or 3. Specific examples of the natural mutant include mutants based on polymorphisms such as SNPs (single nucleotide polymorphisms), and splicing mutants (splicing variants). The substitution is preferably conservative amino acid substitution. This is because the conservative amino acid substitution allows the resulting protein to have a structure or properties substantially equivalent to the APOA2 protein having the amino acid sequence described above. The conservative amino acids refers to the relationship among amino acids classified into the same amino acid groups. For example, a nonpolar amino acid group (glycine, alanine, phenylalanine, valine, leucine, isoleucine, methionine, proline, and tryptophan), a polar amino acid group (amino acids except for the nonpolar amino acids), a charged amino acid group (acidic amino acids (aspartic acid and glutamic acid) and a basic amino acid group (arginine, histidine, and lysine)), an uncharged amino acid group (amino acids except for the charged amino acids), an aromatic amino acid group (phenylalanine, tryptophan, and tyrosine), a branched amino acid group (leucine, isoleucine, and valine), and an aliphatic amino acid group (glycine, alanine, leucine, isoleucine, and valine) are known as the amino acid groups.

The "fragments thereof' refer to fragments of various APOA2 protein variants and their natural mutants, comprising the C-terminal regions of the APOA2 protein variants and the mutants. Specifically, the fragments thereof correspond to protease digestion products of various APOA2 protein variants and their mutants.

The present disclosure provides anti-APOA2 protein terminus antibodies including an anti-APOA2-ATQ terminus antibody and an anti-APOA2-AT terminus antibody.

The "anti-APOA2-ATQ terminus antibody" refers to an antibody capable of specifically recognizing and binding to an epitope present in the C-terminal region of the APOA2-ATQ protein, or a fragment thereof. The phrase "specifically recognizing and binding" means that the antibody can neither recognize nor bind to or hardly recognizes and binds to the other APOA2 protein variants because of no or very weak cross-reactivity with the APOA2 protein variants. Specifically, the anti-APOA2-ATQ terminus antibody refers to an antibody that specifically binds to the C-terminal region of the APOA2-ATQ protein, but exhibits no binding to the C-terminal region of the APOA2-AT protein and the C-terminal region of the APOA2-A protein, etc. Such an antibody directed to the terminus may be any of polyclonal and monoclonal antibodies or fragments thereof. A monoclonal antibody is preferred for achieving large-scale production and for obtaining homogeneous effects.

On the other hand, the "anti-APOA2-AT terminus antibody" refers to an antibody capable of specifically recognizing and binding to an epitope present in the C-terminal region of the APOA2-AT protein, or a fragment thereof. Specifically, the anti-APOA2-AT terminus antibody refers to an antibody that specifically binds to the C-terminal region of the APOA2-AT protein, but exhibits no binding to the C-terminal region of the APOA2-ATQ protein and the C-terminal region of the APOA2-A protein, etc. Such an antibody directed to the terminus may be any of polyclonal and monoclonal antibodies or fragments thereof. A monoclonal antibody is preferred for achieving large-scale production and for obtaining homogeneous effects.

The present disclosure further provides an "anti-APOA2 protein non-terminus antibody" recognizing an amino acid sequence other than the C-terminal region of the APOA2 protein.

The "anti-APOA2 protein non-terminus antibody" refers to an anti-APOA2 antibody recognizing and binding to an epitope present in a region other than the C-terminal region in the full-length amino acid sequence of each APOA2 protein variant. Specifically, the anti-APOA2 protein non-terminus antibody totally differs from the anti-APOA2 protein terminus antibodies in epitope recognized thereby. The term "non-terminus" for the anti-APOA2 protein non-terminus antibody is used for the sake of convenience with respect to the anti-APOA2 protein terminus antibodies. Thus, its epitope is not particularly limited as long as the epitope is present in a region other than the C-terminal region. The anti-APOA2 protein non-terminus antibody can also include an antibody recognizing and binding to an epitope present in the N terminus.

The anti-APOA2 protein non-terminus antibody used in the present invention is preferably an antibody that has almost the same levels of binding activity against an APOA2 protein having a certain C-terminal sequence and binding activity against an APOA2 protein having a C-terminal sequence different from that of the APOA2 protein when the binding activity is compared between these APOA2 proteins, and does not inhibit the binding of the anti-APOA2 protein terminus antibodies to the C-terminal regions. Specific examples thereof include an "anti-APOA2-ATQ non-terminus antibody" binding to an amino acid sequence other than the C-terminal region of the APOA2-ATQ protein shown in SEQ ID NO: 1, an "anti-APOA2-AT non-terminus antibody" binding to an amino acid sequence other than the C-terminal region of the APOA2-AT protein shown in SEQ ID NO: 2. In this case, the antibodies have the same levels of binding activity against these APOA2 proteins, and any of the antibodies do not inhibit the binding of the anti-APOA2-ATQ terminus antibody and the anti-APOA2-AT terminus antibody to the C-terminal regions of the APOA2 proteins. The anti-APOA2 protein non-terminus antibody may be any of polyclonal and monoclonal antibodies or fragments thereof. A monoclonal antibody is preferred for achieving large-scale production and for obtaining homogeneous effects.

The "monoclonal antibody" used in the present specification refers to an antibody that consists of a single immunoglobulin or comprises framework regions (hereinafter, referred to as "FRs") and complementarity determining regions (hereinafter, referred to as "CDRs") and is capable of specifically recognizing and binding to a particular antigen (epitope).

The typical immunoglobulin molecule is a tetramer constituted by two polypeptide chain pairs, i.e., two heavy-light chain pairs, in which the heavy chain in each pair is linked to its partner light chain through a disulfide bond. Each heavy chain is composed of a heavy chain variable region (H chain V region; hereinafter, referred to as "VH") on the N-terminal side and a heavy chain constant region (H chain C region; hereinafter, referred to as "CH") on the C-terminal side. Each light chain is composed of a light chain variable region (L chain V region; hereinafter, referred to as "VL") on the N-terminal side and a light chain constant region (L chain C region; hereinafter, referred to as "CL") on the C-terminal side. Of these regions, VH and VL are particularly important because of their involvement in the binding specificity of the antibody. These VH and VL regions each consist of approximately 110 amino acid residues and internally have three CDRs (CDR1, CDR2, and CDR3) involved directly in the binding specificity for the antigen and four FRs (FR1, FR2, FR3, and FR4) functioning as the backbone structures of the variable region. The CDRs are known to be conformationally complementary to the antigen molecule and to determine the specificity of the antibody (E.A. Kabat et al., 1991, Sequences of proteins of immunological interest, Vol. 1, eds. 5, NIH publication). The amino acid sequences of the constant regions rarely vary among intraspecific antibodies, whereas the amino acid sequences of the CDRs are highly variable among antibodies and, hence, are also called hypervariable regions. In the variable region, the CDRs and the FRs are arranged in the order of FR1, CDR1, FR2, CDR2, FR3, CDR3, and FR4 from the N terminus toward the C terminus. In the immunoglobulin molecule, VL and VH are paired by dimerization to form an antigen-binding site. The immunoglobulin is known to have each class of IgG, IgM, IgA, IgE, and IgD. The antibody may be of any class. IgG is preferred.

The anti-APOA2-ATQ terminus monoclonal antibody specifically binds to the C-terminal region of the APOA2-ATQ protein shown in SEQ ID NO: 1, but exhibits no binding activity against the APOA2-AT protein shown in SEQ ID NO: 2 and the APOA2-A protein shown in SEQ ID NO: 3. Specific examples of such an antibody include anti-APOA2-ATQ terminus monoclonal antibody clones represented by antibody clone names 7F2 and 6G2 described in Example 1 mentioned later. The clone 7F2 has CDR1 consisting of the sequence represented by SEQ ID NO: 4, CDR2 consisting of the sequence represented by SEQ ID NO: 5, and CDR3 consisting of the sequence represented by SEQ ID NO: 6 in a heavy chain, and CDR1 consisting of the sequence represented by SEQ ID NO: 7, CDR2 consisting of the sequence represented by SEQ ID NO: 8, and CDR3 consisting of the sequence represented by SEQ ID NO: 9 in a light chain. The clone 6G2 has CDR1 consisting of the sequence represented by SEQ ID NO: 10, CDR2 consisting of the sequence represented by SEQ ID NO: 11, and CDR3 consisting of the sequence represented by SEQ ID NO: 12 in a heavy chain, and CDR1 consisting of the sequence represented by SEQ ID NO: 13, CDR2 consisting of the sequence represented by SEQ ID NO: 14, and CDR3 consisting of the sequence represented by SEQ ID NO: 15 in a light chain.

The anti-APOA2 protein non-terminus antibody is preferably an antibody having the same levels of binding activity against the APOA2 protein variants shown in SEQ ID NOs: 1 to 3 when the binding activity is compared among them. Specific examples thereof include anti-APOA2 antibody clones represented by antibody clone names MAB1 and MAB2 described in Example 5 mentioned later. The clone MAB1 has CDR1 consisting of the sequence represented by SEQ ID NO: 16, CDR2 consisting of the sequence represented by SEQ ID NO: 17, and CDR3 consisting of the sequence represented by SEQ ID NO: 18 in a heavy chain, and CDR1 consisting of the sequence represented by SEQ ID NO: 19, CDR2 consisting of the sequence represented by SEQ ID NO: 20, and CDR3 consisting of the sequence represented by SEQ ID NO: 21 in a light chain. The clone MAB2 has CDR1 consisting of the sequence represented by SEQ ID NO: 22, CDR2 consisting of the sequence represented by SEQ ID NO: 23, and CDR3 consisting of the sequence represented by SEQ ID NO: 24 in a heavy chain, and CDR1 consisting of the sequence represented by SEQ ID NO: 25, CDR2 consisting of the sequence represented by SEQ ID NO: 26, and CDR3 consisting of the sequence represented by SEQ ID NO: 27 in a light chain. The anti-APOA2-ATQ non-terminus antibody or the anti-APOA2-AT non-terminus antibody can be used as the anti-APOA2 protein non-terminus antibody.

The "fragments thereof' for the "polyclonal and monoclonal antibodies or fragments thereof' are partial fragments of the polyclonal and monoclonal antibodies and refer to polypeptide chains having activity substantially equivalent to the antigen-specific binding activity of the antibodies, or complexes thereof. The fragments each correspond to an antibody portion containing at least one antigen-binding site mentioned above, i.e., a polypeptide chain having at least one VL-VH pair, or a complex thereof. Specific examples thereof include a large number of sufficiently characterized antibody fragments resulting from the cleavage of an immunoglobulin with various peptidases. More specific examples thereof include Fab, F(ab')$_2$, and Fab'. The Fab is a fragment resulting from the papain cleavage of the IgG molecule on the N-terminal side of the disulfide bonds in the hinges and is constituted by a polypeptide consisting of VH and CH1, which is adjacent to the VH, among the three CH-constituting domains (CHI, CH2, and CH3), and a light chain. The F(ab')$_2$ is a Fab' dimer resulting from the pepsin cleavage of the IgG molecule on the C-terminal side of the disulfide bonds in the hinges. The Fab' is substantially structurally equivalent to Fab, though being slightly longer at H chain than Fab by including a hinge (Fundamental Immunology, Paul ed., 3rd ed., 1993). The Fab' can be obtained by reducing F(ab')$_2$ under mild conditions and cleaving the disulfide bridges in the hinge region. All of these antibody fragments contain the antigen-binding site and have the ability to specifically bind to the antigen (i.e., a particular APOA2 protein variant in the present invention).

The fragment of the monoclonal antibody may be synthesized chemically or by use of a recombinant DNA method. Examples thereof include antibody fragments newly synthesized using the recombinant DNA method. Specifically, the fragment corresponds to, but is not limited to, a monomeric polypeptide molecule in which one or more VLs and one or more VHs of the monoclonal antibody are artificially linked via a linker peptide or the like having an appropriate length of a sequence, or a multimeric polypeptide thereof. Examples of such a polypeptide include synthetic antibodies such as single-chain Fv (scFv: single chain fragment of variable region) (see Pierce catalog and Handbook, 1994-1995, Pierce Chemical co., Rockford, IL), diabody, triabody, and tetrabody. In the immunoglobulin molecule, VL and VH are normally positioned on separate polypeptide chains (L chain and H chain). The single-chain Fv is a synthetic antibody fragment having a structure where these variable regions are linked via a flexible linker having a sufficient length such that the VL and the VH are contained in one polypeptide chain. Both of the variable regions in the single-chain Fv are self-assembled with each other to form one functional antigen-binding site. The single-chain Fv can be obtained by integrating a recombinant DNA encoding the single-chain Fv into the phage genome using a technique known in the art, followed by expression. The diabody is a molecule having a structure based on the dimeric structure of the single-chain Fvs (Holliger et al., 1993, Proc. Natl. Acad. Sci USA, 90: 6444-6448). For example, when the linker has a length shorter than approximately 12 amino acid residues, the two variable sites in the single-chain Fv cannot be self-assembled. By contrast, VL in one Fv chain can be assembled with VH in another Fv chain by the formation of the diabody, i.e., by the interaction between the two single-chain Fvs. As a result, two functional antigen-binding sites can be formed (Marvin et al., 2005, Acta Pharmacol. Sin., 26: 649-658). The further addition of cysteine residues to the C termini of the single-chain Fvs permits a disulfide bond between these two Fv chains so that stable diabody can be formed (Alafsen et al., 2004, Prot. Engr. Des. Sel., 17: 21-27). Although the diabody is a divalent antibody fragment as described above, its antigen-binding sites do not have to bind to the same epitope and may have bispecificity of recognizing and specifically binding to different epitopes, respectively. The triabody or the tetrabody has a trimeric or tetrameric structure based on the single-chain Fv structure, as in the diabody. The triabody and the tetrabody are trivalent and quadrivalent antibody fragments, respectively, and may each be a multispecific antibody. The antibody fragment further includes antibody fragments identified using a phage display library (see e.g., McCafferty et al., 1990, Nature, Vol. 348, 552-554), wherein these antibody fragments have the ability to bind to their antigens. Also see, for example, Kuby, J., Immunology, 3rd Ed., 1998, W.H. Freeman & Co., New York.

For use in the present invention, each anti-APOA2 antibody or a fragment thereof can be modified. In this context, the modification includes any of functional modifications (e.g., glycosylation) required for the anti-APOA2 antibody or the fragment thereof to have specific binding activity against the APOA2 protein, and labeling necessary for detecting the antibody or the fragment thereof. Examples of the antibody labeling include labeling with fluorescent dyes (FITC, rhodamine, Texas Red, Cy3, and Cy5), fluorescent proteins (e.g., PE, APC, and GFP), enzymes (e.g., horseradish peroxidase, alkaline phosphatase, and glucose oxidase), or biotin or (strept)avidin. The antibody glycosylation may be altered in order to adjust the affinity of the antibody for the antigen. Such alteration can be achieved, for example, by changing one or more glycosylation sites in the antibody sequence. To be more specific, one or more amino acid substitutions can be introduced to an amino acid sequence constituting one or more glycosylation sites, for example, in FR, to remove the glycosylation sites. As a result, the glycosylation of the sites can be canceled. Such deglycosylation is effective for increasing the affinity of the antibody for the antigen (U.S. Patent Nos. 5714350 and 6350861).

1-2. Preparation of immunogen

In the case of preparing the anti-APOA2 protein terminus antibodies, each APOA2 protein variant is first prepared as an immunogen (antigen). Examples of the APOA2 protein variant that can be used as an immunogen include APOA2 proteins having an amino acid sequence represented by any of SEQ ID NOs: 1 to 3 and mutants thereof, and polypeptide fragments of the proteins or the mutants, and their fusion polypeptides with other peptides (e.g., signal peptides and labeling peptides). The APOA2 protein variant as an immunogen can be synthesized by an approach known in the art, for example, a solid-phase peptide synthesis method, for example, using information on the amino acid sequence represented by any of SEQ ID NOs: 1 to 3. The APOA2 protein variant can be prepared by, for example, a method given below.

Any of naturally occurring APOA2 proteins and recombinant APOA2 proteins can be used as the APOA2 protein variant. A synthetic APOA2 protein, the whole or a portion of which has been chemically synthesized by peptide synthesis or the like may be used as an immunogen. For example, the APOA2 protein variant that is prepared in order to obtain each antibody binding to the APOA2 protein C terminus (anti-APOA2 protein terminus antibody) may be any of naturally occurring APOA2 proteins, recombinant APOA2 proteins, and synthetic APOA2 proteins, the whole or a portion of which has been chemically synthesized by peptide synthesis or the like, comprising an amino acid sequence consisting of at least 6 or more consecutive amino acids of the C-terminal regions of various APOA2 protein variants.

The naturally occurring APOA2 proteins can be recovered from samples including body fluids such as blood (including serum and plasma), or culture supernatants of cultured cells by use of a protein separation and purification technique known in the art, for example, gel filtration, ion-exchange chromatography, or affinity chromatography.

The recombinant APOA2 proteins can be expressed in microbes, insect cells, or animal cells harboring DNAs encoding the proteins and then recovered from the cells by use of a protein separation and purification technique known in the art.

The synthetic APOA2 proteins can be synthesized by an approach known in the art, for example, a solid-phase peptide synthesis method, for example, using published information on the amino acid sequence of the APOA2 protein. These synthetic APOA2 proteins may each be linked to a carrier protein such as KLH (keyhole limpet hemocyanin), OVA (ovalbumin), or BSA (bovine serum albumin).

In the case of using a fragment of the APOA2 protein variant as an immunogen in the anti-APOA2 protein terminus antibody preparation, any of naturally occurring APOA2 protein fragments, recombinant APOA2 protein fragments, and synthetic APOA2 protein fragments can also be used. For example, an oligopeptide or a polypeptide comprising 6 or more, preferably 10 or more, more preferably 18 or more, further preferably 30 or more consecutive amino acid residues including the C terminus of a sequence represented by any of SEQ ID NOs: 1 to 3 can be used as the APOA2 protein fragment serving as an antigen. For example, a peptide comprising the amino acid sequences represented by SEQ ID NO: 28 or 29 can be used.

In the case of using a fragment of the naturally occurring APOA2 protein as an immunogen, for example, in the anti-APOA2 protein terminus antibody preparation, the purified APOA2 protein is treated with suitable protease such as trypsin and then fractionated on a reverse-phase column to obtain peaks. The amino acid sequence of the peptide contained in each peak is determined with a mass spectrometer. The peak of the peptide comprising, as a partial sequence, a sequence consisting of 6 or more consecutive amino acids of the C-terminal region of the APOA2 protein shown in any of SEQ ID NOs: 1 to 3 can be used as the immunogen.

In the case of using a partial amino acid sequence of the recombinant APOA2 protein as an immunogen, for example, in the anti-APOA2 protein terminus antibody preparation, a DNA sequence portion encoding a partial sequence of 6 or more consecutive amino acids including the C-terminal amino acid residues of the APOA2 protein shown in any of SEQ ID NOs: 1 to 3, in a DNA sequence encoding the APOA2 protein mentioned above, can be inserted to vectors for expression, which are then transferred to various cells to obtain the partial amino acid sequence of each APOA2 protein variant represented by any of SEQ ID NOs: 1 to 3.

Also in the case of preparing the anti-APOA2 protein non-terminus antibody, its preparation method can be basically the same as the method for preparing the anti-APOA2 protein terminus antibodies except that a region that can be used as an immunogen in the APOA2 protein is different from the regions used as an immunogen for preparing the anti-APOA2 protein terminus antibodies. Specifically, the whole or a portion of a region other than the C-terminal region of the APOA2 protein can be used as an immunogen. In the case of preparing the anti-APOA2 protein non-terminus antibody, as in the case of preparing the anti-APOA2 protein terminus antibodies, an oligopeptide or a polypeptide comprising amino acid residues of the region other than the C-terminal region of the APOA2 protein can also be used as an antigen.

(Preparation of recombinant APOA2 protein)

Hereinafter, the preparation of a recombinant APOA2 protein (recombinant APOA2 protein variant) shown in any of SEQ ID NOs: 1 to 3 will be described in detail.

(a) Preparation of polynucleotide encoding recombinant APOA2 protein variant

Phages or plasmids capable of autonomously replicating in host microbes can be used as vectors for use in the expression of various APOA2 protein variants. Examples of the plasmids include *E. coli*-derived plasmids (pET30a, pGEX6p, pUC118, pUC119, pUC18, pUC19, etc.), *Bacillus subtilis*-derived plasmids (pUB110, pTP5, etc.), and enzyme-derived plasmids (YEp13, YEp24, YCp50, etc.). Examples of the phages include λ phages (λgt11, λZAP, etc.). In addition, vectors of animal viruses such as vaccinia virus or insect viruses such as baculovirus can also be used.

The method for inserting a polynucleotide encoding the APOA2 protein variant to the vectors involves, for example, cleaving the purified polynucleotide with appropriate restriction enzymes and ligating the resulting fragment into the vectors cleaved with the appropriate restriction enzymes by use of DNA ligase or the like.

(b) Transfer of APOA2 protein variant expression vector into host

The obtained APOA2 protein variant expression vectors are transferred to hosts capable of expressing the expression vectors to obtain APOA2 protein variant-expressing transformants. The hosts used are not particularly limited as long as the hosts are suitable for the vectors used and are capable of expressing the APOA2 protein variant. For example, bacteria (*E. coli* (*Escherichia coli*), *Bacillus subtilis,* yeasts, insect cells, or animal cells (COS cells and CHO cells (Journal of immunology, 1998, Vol. 160, 3393-3402)) are preferably used. The method for transferring the vectors to the bacteria is not particularly limited as long as the method is a method known in the art for transferring the vectors to the bacteria. Examples thereof include a heat shock method, a method using calcium ions, and electroporation. All of these techniques are known in the art and described in various literatures. See, for example, Greene & Sambrook, 2012, Molecular Cloning: A Laboratory Manual Fourth Ed., Cold Spring Harbor Laboratory Press, Cold Spring Harbor, New York. A Lipofectin method (PNAS, 1989, Vol. 86, 6077; and PNAS, 1987, Vol. 84, 7413), electroporation, a calcium phosphate method (Virology, 1973, Vol. 52, 456-467), a DEAE-dextran method or the like is preferably used in the transformation of the animal cells.

In the case of using bacteria as the hosts, preferably, the APOA2 protein variant expression vectors are capable of autonomously replicating in the bacteria and are also constituted by a promoter sequence, a ribosomal binding sequence, the DNA sequence encoding the APOA2 protein variant, and a transcription termination sequence. The expression vectors may also contain a gene encoding a regulatory factor controlling the promoter. Any promoter that can function in the hosts such as *E. coli* may be used.

Likewise, in the case of using eukaryotic cells such as yeasts, animal cells, or insect cells as the hosts, APOA2 protein variant-expressing transformants can also be obtained according to an approach known in the art. The APOA2 protein variant expression vectors for use in the eukaryotic cells contain a promoter sequence and the DNA sequence encoding the APOA2 protein variant, which may be linked, if desired, to a cis element (e.g., an enhancer), a splicing signal (a donor site, an acceptor site, a branch point, etc.), a poly-A addition signal, a selective marker sequence, a ribosomal binding sequence (SD sequence), and the like.

(c) Culture of transformant and expression of recombinant APOA2 protein variant

Subsequently, the prepared transformants are cultured. The method for culturing the transformants in a medium is carried out according to an ordinary method for use in the culture of the hosts. In the case of using, for example, bacteria as the hosts, the medium is not particularly limited as long as the medium contains a carbon source, a nitrogen source, inorganic salts, etc., utilizable by the bacteria and the bacteria are capable of growing or proliferating in the medium. Any of natural and synthetic media can be used. More specific examples thereof include an LB medium, but are not limited thereto, as a matter of course. For the selective culture of the transformants, an antibiotic such as ampicillin or tetracycline may be added to the medium, if necessary. The culture is usually carried out at 37°C for 6 to 24 hours under aerobic conditions such as culture with aeration and stirring. During the culture period, the pH is preferably kept at or around a neutral pH. The pH is adjusted using an inorganic or organic acid or alkali solution or the like. When the transformants are animal cells such as CHO cells, the host cells can be inoculated at $1 \times 10^5$ cells/mL to a DMEM medium manufactured by Gibco/Thermo Fisher Scientific, Inc. and cultured in a 5% $CO_2$ incubator of 37°C. During the culture, an antibiotic such as ampicillin or tetracycline may be added to the medium, if necessary.

When the APOA2 protein variant expression vectors are protein expression induction-type vectors containing a protein expression control system (which corresponds to, for example, a repressor gene and an operator for the host bacteria), the expression of the APOA2 protein variant needs to be induced by a predetermined treatment of the transformants. The expression induction method differs depending on the protein expression control system contained in the vectors. Therefore, induction treatment suitable for the system can be carried out. For example, the protein expression control system most generally used in the protein expression induction-type vectors for use in the host bacteria is a system consisting of a lac repressor gene and a lac operator. This system is capable of inducing the expression by IPTG (isopropyl-1-thio-β-D-galactoside) treatment. The transformants having the APOA2 protein expression vectors containing this system can be allowed to express the APOA2 protein variant of interest by the addition of IPTG in an appropriate amount (e.g., final concentration: 1 mM) into the medium.

(d) Extraction and/or recovery of recombinant APOA2 protein variant

When the APOA2 protein variant is produced inside the bacterial bodies or the cells after the culture, the bacterial bodies or the cells can be recovered and disrupted, followed by protein extraction. When the APOA2 protein variant is produced outside the bacterial bodies or the cells, the culture solution can be used directly, or the supernatant obtained by the removal of the bacterial bodies or the cells through centrifugation or the like can be used. Then, the APOA2 protein variant can be isolated and purified from the cultures by using, alone or in appropriate combination, general protein purification methods, for example, ammonium sulfate precipitation, gel filtration, ion-exchange chromatography, and affinity chromatography. Whether or not the APOA2 protein variant has been obtained can be confirmed by SDS-polyacrylamide gel electrophoresis or the like.

1-3. Preparation of anti-APOA2 monoclonal antibody

1-3-1. Methods for preparing anti-APOA2 monoclonal antibody and hybridoma

Hybridomas producing the anti-APOA2 monoclonal antibody can be prepared by a method described below. However, the preparation method is not limited thereto, and any of other methods known in the art can be used for the preparation.

(1) Method for preparing anti-APOA2 monoclonal antibody

In order to prepare the anti-APOA2 protein terminus monoclonal antibody specifically binding to the C-terminal region of any APOA2 protein shown in SEQ ID NO: 1, 2, or 3 in the amino acid sequence constituting the APOA2 protein, monoclonal antibodies can be prepared with the APOA2 protein variant or a peptide comprising the C-terminal region of the APOA2 protein variant as an immunogen and then screened for an antibody binding only to the particular APOA2 protein variant using the APOA2 protein shown in any of SEQ ID NOs: 1 to 3 or the peptide comprising the C-terminal region of the APOA2 protein variant. For example, the anti-APOA2-ATQ terminus monoclonal antibody can be selected by screening using, as an index, specific binding to the C-terminal region of the APOA2-ATQ protein shown in SEQ ID NO: 1 without or almost without binding to the APOA2 protein variant shown in SEQ ID NO: 2 or 3. Also, the anti-APOA2-AT terminus monoclonal antibody can be selected by screening using, as an index, specific binding to the C-terminal region of the APOA2-AT protein shown in SEQ ID NO: 2 without or almost without binding to the APOA2 protein variant shown in SEQ ID NO: 1 or 3.

In order to prepare the anti-APOA2 protein non-terminus antibody recognizing amino acids other than the C-terminal region of the APOA2 protein, monoclonal antibodies can be prepared with the APOA2 protein variant or a peptide comprising a partial sequence thereof as an immunogen and then screened for the antibody of interest by using, as an index, the same levels of binding activity against the APOA2 protein variants shown in SEQ ID NOs: 1 to 3 or their peptides differing in C terminus when the binding activity is compared among them.

(2) Preparation of anti-APOA2 antibody-producing cell

The recombinant APOA2 protein obtained as an immunogen in the paragraph 1-2 is dissolved in a buffer solution to prepare an immunogen solution. For effective immunization, an adjuvant may be added thereto, if necessary. Examples of the adjuvant include commercially available Freund's complete adjuvant (FCA) and Freund's incomplete adjuvant (FIA). These adjuvants may be used alone or as a mixture.

Next, a mammal, for example, a rat, a mouse (e.g., an inbred mouse BALB/c), or a rabbit is immunized by the administration of the prepared immunogen solution. Examples of the immunogen administration method include, but are not limited to, subcutaneous injection using FIA or FCA, intraperitoneal injection using FIA, and intravenous injection using 0.15 mol sodium chloride. One dose of the immunogen is appropriately determined according to the type of the animal to be immunized, an administration route, etc., and is approximately 50 to 200 µg per animal. The intervals between the immunization shots are not particularly limited. After the priming, 2 to 6, preferably 3 or 4 boosters are performed at intervals of a few days to a few weeks, preferably at 1 - to 4-week intervals. After the priming, an antibody titer in the serum of the immunized animal is measured by ELISA (enzyme-linked immunosorbent assay) or the like. Provided that a sufficient rise in antibody titer is confirmed, the immunogen is intravenously or intraperitoneally injected for final immunization. 2 to 5 days, preferably 3 days, after the final immunization date, antibody-producing cells are collected.

1-3-2. Method for preparing anti-APOA2 monoclonal antibody-producing hybridoma

(1) Recovery of antibody-producing cell from immunized animal and cell fusion

The antibody-producing cells obtained from the immunized animal can be subjected to cell fusion with myeloma cells to prepare hybridomas producing the monoclonal antibody specifically recognizing the particular region of the APOA2 protein. Examples of the antibody-producing cells include spleen cells, lymph node cells, and peripheral blood cells. Spleen cells or local lymph node cells are preferred. A generally available established cell line derived from mice or the like can be used as the myeloma cells for fusion with the antibody-producing cells. The cell line used preferably has drug selectivity and has the property of being unable to survive in an unfused state in a HAT selective medium (containing hypoxanthine, aminopterin, and thymidine) and being able to grow therein only in a state fused with the antibody-producing cells. Also, the established cell line is preferably derived from an animal of the same species as the immunized animal. Specific examples of the myeloma cells include BALB/c mouse-derived hypoxanthine-guanine-phosphoribosyl-transferase (HGPRT)-deficient cell lines P3X62-Ag.8 (ATCCTIB9), P3X63-Ag.8.UI (JCRB9085), P3/NSI/1-Ag4-1 (JCRB0009), P3x63Ag8.653 (JCRB0028), and SP2/0-Agl4 (JCRB0029).

For the cell fusion between the myeloma cells and the antibody-producing cells, the antibody-producing cells and the myeloma cells are mixed at a ratio of approximately 1:1 to 20:1 in a serum-free medium for animal cell culture, such as a DMEM or RPMI1640 medium, and fused with each other through reaction in the presence of a cell fusion promoter. For example, polyethylene glycol having an average molecular weight of 1,500 to 4,000 Da can be used as the cell fusion promoter at a concentration of approximately 10 to 80%. If necessary, an auxiliary such as dimethyl sulfoxide may be used in combination therewith for enhancing fusion efficiency. Alternatively, the antibody-producing cells and the myeloma cells may be fused with each other using a commercially available cell fusion apparatus that employs electric stimulation (e.g., electroporation) (Nature, 1977, Vol. 266, 550-552).

(2) Selection of hybridoma of interest

In a method for selecting hybridomas producing the anti-APOA2 monoclonal antibody of interest from the cells after the cell fusion treatment, the cell suspension is appropriately diluted with, for example, an RPMI1640 medium containing fetal bovine serum and then seeded at approximately $2 \times 10^6$ cells/well over a 96-well microtiter plate. A selective medium is added to each well where the cells are subsequently cultured with the selective medium appropriately replaced with a fresh one. The culture temperature is 20 to 40°C, preferably approximately 37°C. When the myeloma cells are of HGPRT-deficient line or thymidine kinase (TK)-deficient line, only hybridomas of the antibody-producing cells and the myeloma cells can be selectively allowed to grow or proliferate by use of a selective medium containing hypoxanthine, aminopterin, and thymidine (HAT medium). Therefore, cells grown from approximately 10 days after the start of culture in the selective medium can be selected as the hybridomas.

The hybridomas selected in the HAT medium are first screened by using binding activity against various APOA2 protein variants shown in SEQ ID NOs: 1 to 3 as an index. Subsequently, the hybridomas producing the antibody having binding activity against the APOA2 protein variant are tested for cross-reactivity to select acceptable ones. The acceptable cross-reactivity means cross-reactivity at a negligible level for the intended purposes of the antibody. For example, a monoclonal antibody for use in immunological assay can be regarded as having practically no cross-reactivity when signal intensity from cross reaction in a final assay system can be suppressed at a background level to less than 1% of signal intensity from specific reaction.

For example, ELISA can be used for confirming reaction specificity for the particular APOA2 protein variant. In this ELISA method, a microplate in which various APOA2 protein variants or fragments thereof are separately immobilized as antigens on different wells is prepared and reacted by the addition of appropriately diluted samples of the culture supernatant of the hybridomas. After sufficient reaction, the wells are washed and further reacted by the addition of a labeled form of a secondary antibody directed to an immunoglobulin. The wells are washed again and can be finally assayed by use of the label of the secondary antibody bound with the wells to quantitatively determine the binding activity of the antibody present in the culture supernatant against the antigens. For example, for the preparation of the anti-APOA2 protein terminus monoclonal antibody, the specificity can be determined by using, as an index, the exhibition of binding activity only against the C-terminal region of the particular APOA2 protein variant without cross-reactivity with the other APOA2 protein variants. For the preparation of the anti-APOA2 protein non-terminus monoclonal antibody, the antibody is selected by using, as an index, the same levels of binding activity against all of the APOA2 protein variants differing in C terminus without the inhibition of the binding of the anti-APOA2 protein terminus monoclonal antibody to the C-terminal region by the prepared antibody.

The hybridomas can also be selected by use of a recombinant DNA technique. First, mRNAs are extracted from the hybridoma group obtained according to the aforementioned method. The mRNA extraction can be carried out by use of a method known in the art. Subsequently, cDNAs are obtained from the mRNAs using Oligo dT primers or random primers. The cDNAs are used as templates in PCR using primer sets comprising the nucleotide sequence of the signal sequence upstream of the variable region-encoding gene and a nucleotide sequence on the constant region side. The obtained amplification products can be inserted to appropriate cloning vectors and cloned to obtain a library of the variable region genes of the antibodies produced by the hybridomas. As a more specific non-limiting example, PCR is carried out using Mouse Ig Primer provided by Novagen/Merck KGaA, and the amplification products (mouse immunoglobulin variable region cDNAs) are inserted to the EcoRI sites of ZERO BLUNT PCR TOPO Vectors provided by Invitrogen Corp. and cloned. The obtained vector group can be used as a library of the genes encoding the variable region amino acid sequences. Next, a probe is designed on the basis of the amino acid sequence of each variable region or each CDR disclosed in the present disclosure. The library can be screened for positive clones to select the hybridomas producing the antibody of the present disclosure.

(3) Antibody production using hybridoma

The hybridomas can be used in antibody production by ascites formation using a mouse. Specifically, the hybridomas are intraperitoneally inoculated to a mouse of the origin of the fusion partner cells used for preparing the hybridomas, or to a nude mouse. The ascites can be appropriately collected to recover an antibody-containing ascites fluid. More specifically, hybridomas obtained with SP2/0 cells as a fusion partner are intraperitoneally inoculated to a BALB/c mouse after a lapse of 10 days after inoculation with pristine, and an antibody-containing ascites fluid can be recovered.

The hybridomas can be used in antibody production by culture using a suitable medium. Specifically, the hybridomas can be inoculated at $1 \times 10^5$ cells/mL into a Hybridoma-SFM medium manufactured by Gibco/Thermo Fisher Scientific, Inc. and cultured in a 5% $CO_2$ incubator of 37°C until the hybridomas are killed to obtain an antibody-containing culture supernatant, though the antibody production method according to the present disclosure is not limited thereto.

(4) Method for preparing recombinant anti-APOA2 monoclonal antibody or fragment thereof by recombinant DNA manipulation

The antibody or the fragment thereof can also be obtained by recombinant DNA manipulation using a cDNA sequence encoding the amino acid sequence of the antibody.

Nucleotide sequences encoding the amino acid sequences of the variable regions of the antibody derived from an anti-APOA2 monoclonal antibody-producing hybridoma, for example, the anti-APOA2 protein terminus monoclonal antibody-producing hybridoma obtained by the approach described in the paragraph "1-3-2(2)", are used. These nucleotide sequences of VH and VL are linked to nucleotide sequences encoding arbitrary CH and CL, respectively, and the resulting polynucleotides can be incorporated into appropriate expression vectors, which are then transferred to host cells, followed by expression as a complete immunoglobulin molecule. Alternatively, according to a CDR grafting antibody technique, polynucleotides encoding the amino acid sequences of the CDR sequences in the amino acid sequences of the variable regions obtained by the approach described in the paragraph "1-3-2(2)" may be incorporated into appropriate expression vectors, which are then transferred to host cells, followed by expression as a complete immunoglobulin molecule. In this respect, an approach is convenient in which the heavy chain and the light chain to be paired can be expressed in the same host cell and produced as a heavy chain/light chain dimer. Specifically, each cell is cotransfected with, for example, the light chain expression vector and the heavy chain expression vector, and the antibody can also be obtained from this transformed cell. Alternatively, polynucleotides encoding the amino acid sequences described above may be incorporated directly to appropriate expression vectors, which are then transferred to host cells, followed by expression as fragments of the immunoglobulin molecule. Alternatively, as mentioned above, polynucleotides respectively encoding VL and VH or the light chain and the heavy chain comprising the amino acid sequences may be linked via a nucleotide sequence encoding an appropriate linker, then incorporated to phages, and expressed as single-chain Fv or as a synthetic antibody fragment such as diabody. In addition, according to a recently developed phage display antibody technique (Brinkmann et al., 1995, J. Immunol Methods, 182, 41-50; and International Publication Nos. WO97/13844 and WO90-02809), which involves expressing recombinant antibodies on phage surface by exploiting a gene engineering technique, single-chain Fv antibodies diversified by artificially shuffling genes encoding heavy and light chains can be expressed as phage-fusion proteins to obtain specific antibodies.

The methods for preparing the polynucleotide encoding the recombinant anti-APOA2 antibody or the fragment thereof, preparing vectors carrying the polynucleotide, and transferring the vectors to hosts can be carried out by use of a recombinant DNA technique known in the art. The recombinant anti-APOA2 protein antibody of interest or the fragment thereof can be obtained from the culture solution of the transformed cells or from the inside of the cells.

For example, plasmids, phagemids, cosmids, or virus vectors (e.g., SV40 viru based vector, EB virus based vector, and BPV based vector) can be used as immunoglobulin expression vectors, though the vectors are not limited thereto. For example, a BCMGS Neo vector, which is a BPV based vector, is a desirable vector for efficient expression of a foreign gene by the transformation of COS7 cells or the like therewith (Hajime Karasuyama, "Bovine papilloma virus vector", Masami Muramatsu and Hiroto Okayama, ed., Experimental Medicine, Suppl.: Handbook of Gene Engineering, 1991, Yodosha Co., Ltd., 297-299).

The vectors can contain control elements (e.g., a promoter, an enhancer, a terminator, a polyadenylation site, and a splicing site) necessary for expressing the antibody or the fragment thereof, or an optional selective marker, in addition to the polynucleotide encoding the antibody or the fragment thereof.

The hosts described in the paragraph "1-2. Preparation of immunogen" as well as SP2/0 (mouse myeloma) cells (European Journal of Cancer Prevention (1996) Vol. 5, 512-519; and Cancer Research (1990) Vol. 50, 1495-1502) are preferably used as the hosts for transformation.

The host cells containing the vectors for the expression of the antibody or the fragment thereof can be cultured according to a routine method so that the antibody is produced into the culture supernatant or the host cells. Specifically, when the hosts are CHO cells, the host cells can be inoculated at $1 \times 10^5$ cells/mL to a DMEM medium manufactured by Gibco/Thermo Fisher Scientific, Inc. and cultured in a 5% $CO_2$ incubator of 37°C to obtain an antibody-containing culture supernatant. When the host cells are, for example, *E. coli* cells, the host cells can be inoculated to a general medium for use in *E. coli* culture, such as an LB medium, and cultured for the induction of protein expression to produce the antibody into the culture supernatant or the host cells.

The expression product antibody or fragment thereof containing constant regions can be purified and recovered from the culture supernatant or cell lysates using a protein A column, a protein G column, an anti-immunoglobulin antibody affinity column, or the like. By contrast, this purification method cannot be applied to the expression product consisting of variable regions without containing constant regions. Therefore, other appropriate purification methods are applied thereto. The antibody or the fragment thereof can be expressed as a structure C-terminally fused with, for example, a tag sequence advantageous for purification, such as a histidine tag, and thereby purified by affinity chromatography using the corresponding ligand. Unless being the tag-fusion protein, such an antibody or a fragment thereof can be purified according to a routine protein purification method such as ammonium sulfate precipitation, ion-exchange chromatography, reverse-phase chromatography, gel filtration chromatography, or hydroxyapatite chromatography.

In order to confirm specificity for the particular APOA2 protein variant or the fragment thereof, the monoclonal antibody or the fragment thereof used in the present invention is preferably tested for cross-reactivity with the other variants before use, as mentioned above. The antigens for which the cross-reactivity of, for example, the anti-APOA2-ATQ protein terminus monoclonal antibody or the fragment thereof should be confirmed are the APOA2-AT protein and the APOA2-A protein.

It is more preferred to confirm the cross-reactivity of the antibody or the fragment thereof used in the present invention with the proteins described above as well as other proteins having a partial structure in common with the APOA2 protein variants. ELISA with, for example, the APOA2-ATQ protein as an antigen may be used for confirming cross reaction. The antibody to be tested for reaction specificity, i.e., the anti-APOA2 protein terminus antibody or the fragment thereof, is reacted with the APOA2 protein variant in the presence of other antigen proteins for which the cross-reactivity should be confirmed. The cross-reactivity can be confirmed by observing the competition between the APOA2 protein variant and the antigen proteins. Such a method for confirming the cross-reactivity through the use of the principles of competitive inhibition eliminates the need of preparing reaction systems for all antigens and therefore permits rapid screening.

1-3-3. Structural confirmation of region in APOA2 protein recognized by obtained anti-APOA2 protein terminus monoclonal antibody

The type of the APOA2 protein variant specifically recognized by the obtained anti-APOA2 monoclonal antibody can be determined by preparing genes of various APOA2 protein variants using PCR reaction or the like on the basis of the gene of the APOA2 protein, and analyzing the binding activity of the monoclonal antibody against the various APOA2 protein variants obtained from the genes.

In the case of the anti-APOA2 protein terminus monoclonal antibody, such a method is specifically carried out as follows: first, the full-length APOA2 gene or varying lengths of fragments lacking 6 bases or 9 bases including the stop codon of the APOA2 gene from the stop codon toward the 5' end are prepared, and expression vectors having inserts of these fragments are prepared. Such a method for preparing the gene fragments having deletion mutation are described in "Zoku-Seikagaku Jikken Koza 1, Idenshi Kenkyuho II (Experiments in Biochemistry, second series, Methods in Gene research in English), p. 289-305, The Japanese Biochemical Society ed". Next, various APOA2 protein variants are prepared by the aforementioned method from host cells harboring the respective APOA2 protein variant expression vectors. Subsequently, the binding activity of the anti-APOA2 protein monoclonal antibody against the various APOA2 protein variants is evaluated by ELISA using these proteins as antigens. The monoclonal antibody can be determined as an anti-APOA2 protein terminus monoclonal antibody specifically binding to the particular APOA2 protein variant when the monoclonal antibody exhibits binding activity only against the particular variant and exhibits no or almost no binding activity against the other variants.

The APOA2 protein variant recognized by the obtained anti-APOA2 protein terminus monoclonal antibody can also be confirmed by a method as described below.

First, peptides having the sequences of the C-terminal regions of various APOA2 protein variants are each synthesized in a solid phase by a method known in the art. Subsequently, the binding activity of the anti-APOA2 protein terminus monoclonal antibody against the various peptides is evaluated by ELISA using these peptides as antigens. The anti-APOA2 protein monoclonal antibody can be determined as an anti-APOA2 protein terminus monoclonal antibody specifically binding to the particular APOA2 protein variant when the monoclonal antibody is found to have binding activity only against the peptide having the sequence of the particular C-terminal region.

1-4. Preparation of anti-APOA2 polyclonal antibody

The anti-APOA2 polyclonal antibody can be prepared by a method known in the art. Hereinafter, the method for obtaining an anti-APOA2 protein terminus antibody specifically binding to the particular APOA2 protein variant will be specifically given as an example.

1-4-1. Obtainment of antiserum

In order to prepare the anti-APOA2 protein terminus polyclonal antibody, first, a C-terminal fragment having a length of at least 6 or more amino acids on the particular APOA2 protein variant sequence, for example, the peptide shown in SEQ ID NO: 28 or 29, is dissolved in a buffer solution to prepare an immunogen solution. For effective immunization, an adjuvant may be added thereto, if necessary. Examples of the adjuvant include commercially available Freund's complete adjuvant (FCA) and Freund's incomplete adjuvant (FIA). These adjuvants can be used alone or as a mixture.

Next, a mammal, for example, a rat, a mouse (e.g., an inbred mouse Balb/c), or a rabbit is immunized by the administration of the prepared immunogen solution. One dose of the immunogen solution is appropriately determined according to the type of the animal to be immunized, an administration route, etc., and can involve approximately 50 to 200 $\mu$g of the immunogen per animal. Examples of the administration method of the immunogen solution include, but are not limited to, subcutaneous injection using FIA or FCA, intraperitoneal injection using FIA, and intravenous injection using 150 mM sodium chloride. The intervals between the immunization shots are not particularly limited. After the priming, 2 to 10, preferably 3 or 4 boosters are performed at intervals of a few days to a few weeks, preferably at 1- to 4-week intervals. After the priming, an antibody titer in the serum of the immunized animal is repeatedly measured by ELISA (enzyme-linked immunosorbent assay) or the like. Provided that a sufficient rise in antibody titer is observed, the immunogen solution is intravenously or intraperitoneally injected for final immunization. Antiserum containing the polyclonal antibody recognizing the APOA2 protein can be recovered from the blood of the animal thus immunized.

1-4-2. Purification of anti-APOA2 antibody

(1) Preparation of peptide-immobilized column

Affinity columns are prepared by respectively immobilizing the APOA2 protein C-terminal region peptide and a C-terminally amide group-added APOA2 protein C-terminal region peptide. The detailed method is described in "Experimental Protocol for Anti-Peptide Antibodies", the 2nd edition, Gakken Medical Shujunsha Co., Ltd. For example, formyl-Cellulofine or CNBr agarose carriers having functional groups capable of binding to amino groups of peptides, or carriers capable of binding to cysteine residues on peptide sequences via maleimide groups covalently bonded to the carriers can be used as carriers for use in the affinity columns. The length of the peptide to be immobilized is 6 or more amino acids, preferably 10 or more amino acids, more preferably 18 or more amino acids, further preferably 30 or more amino acids, including the C terminus of the APOA2 protein.

(2) Antibody purification

The anti-APOA2 protein terminus polyclonal antibody can be purified from the antiserum using the peptide-immobilized affinity columns. For example, the antiserum is diluted with a suitable buffer solution. IgG antibodies contained in the antiserum are adsorbed onto the APOA2 protein C-terminal region peptide-immobilized affinity column, and this adsorbed fraction is recovered. Subsequently, immunoglobulins exhibiting binding activity against a region other than the C-terminal region of the peptide are removed by adsorption using the C-terminally amidated APOA2 protein peptide-immobilized affinity column. Finally, the resulting non-adsorbed fraction is obtained as an anti-APOA2 protein terminus polyclonal antibody specifically recognizing the particular APOA2 protein variant.

2. Method for detecting pancreatic tumor

The present invention relates to a method for detecting a pancreatic tumor, i.e., pancreatic cancer or benign pancreatic tumor, *in vitro* as defined in the appended claims. The method of the present invention is based on the finding that the amounts of both or one of the two APOA2 protein variants, i.e., the APOA2-ATQ protein and the APOA2-AT protein, in blood are significantly decreased in pancreatic tumor patients compared with normal persons. A feature of this method is to assay the two APOA2 protein variants using antibodies specifically recognizing the C-terminal regions of the APOA2 protein variants (anti-APOA2 protein terminus antibodies) or fragments thereof and anti-APOA2 protein antibodies recognizing the amino acid sequences of regions other than these C-terminal regions (anti-APOA2 protein non-terminus antibodies) or fragments thereof. A further feature of the method for detecting a pancreatic tumor is multivariate analysis using the measurement values of the two APOA2 protein variants assayed.

The method of the present invention comprises the step of assaying the markers for pancreatic tumor detection and the step of determining affection, as defined in the appended claims.

Hereinafter, each step will be described in detail.

2-1. Step of assaying markers for pancreatic tumor detection

The "step of assaying the markers for pancreatic tumor detection" is the step of measuring *in vitro* the amounts of the markers for pancreatic tumor detection, i.e., the two APOA2 protein variants (APOA2-ATQ protein and APOA2-AT protein), present in a body fluid derived from a test subject.

In the present specification, the "test subject" refers to an individual that is subject to the pancreatic tumor detection, preferably an individual suspected of having a pancreatic tumor. In this context, examples of the individual include vertebrates. The individual is preferably a mammal, for example, a primate (a human, a monkey, a chimpanzee, an orangutan, a gorilla, etc.), a rodent (a mouse, a rat, a guinea pig, etc.), or an ungulate animal (cattle, a horse, sheep, a goat, etc.), more preferably a human. In the present specification, when the test subject is a human, this test subject is particularly referred to as a "human test subject" below.

In the present specification, the "body fluid" is a sample that is subjected to the pancreatic tumor detection, and means a biological fluid. The body fluid is not particularly limited as long as the body fluid is a biological fluid possibly containing the markers for pancreatic tumor detection of the present invention. The body fluid includes, for example, blood, urine, lymphocyte culture supernatants, spinal fluid, digestive juice (including e.g., pancreatic juice, large intestine juice, esophageal gland secretions, and saliva), sweat, ascites, runny nose, tear, vaginal fluid, and semen. Blood or urine is preferred. In the present specification, the "blood" includes whole blood, plasma, and serum. The whole blood is not limited by its type and can be, for example, venous blood, arterial blood, or umbilical cord blood. The body fluid may be a combination of two or more different body fluids obtained from the same individual. The method for detecting a pancreatic tumor according to the present invention permits detection even from low invasive blood or urine and is therefore very useful as a convenient detection method.

The "body fluid derived from a test subject" refers to a body fluid already collected from the test subject, and the act of collecting the body fluid is not included in the scope of the present invention. The body fluid derived from a test subject may be subjected to the method of the present invention immediately after being collected from the test subject. Alternatively, the body fluid derived from a test subject may be refrigerated or frozen immediately after collection or after an appropriate treatment and brought to room temperature before being subjected to the method of the present invention. The appropriate treatment before refrigeration or freezing includes, for example, the anticoagulation treatment of whole blood by the addition of heparin or the like, followed by separation as plasma or serum. Such a treatment can be carried out on the basis of a technique known in the art.

In the present specification, the "amounts of the APOA2 protein variants" refer to the respective quantities of the two APOA2 protein variants present in the body fluid derived from a test subject. The quantities may be any of absolute and relative amounts. The absolute amounts correspond to the masses or volumes of the two APOA2 protein variants contained in a predetermined amount of the body fluid. The relative amounts refer to, for example, relative measurement values of the two APOA2 protein variants derived from the test subject to the measurement values of standards used. Examples of the relative amounts include concentrations, fluorescence intensity, and absorbance.

The amounts of the APOA2 protein variants can be measured *in vitro* by use of a method known in the art. Examples thereof include a measurement method using substances capable of specifically binding to the two APOA2 protein variants, respectively.

In the present specification, the term "capable of specifically binding" means that a certain substance can substantially bind only to the particular APOA2 protein variant as a target of the present invention. In this case, the presence of nonspecific binding is acceptable without influencing the detection of the particular APOA2 protein variant.

Examples of the "substances capable of specifically binding" include APOA2-binding proteins. More specifically, the substances capable of specifically binding are, for example, "anti-APOA2 protein terminus antibodies" recognizing the difference in C-terminal region structure and binding to the APOA2 protein variants as their respective antigens, preferably "anti-human APOA2 protein terminus antibodies" each recognizing and binding to only one of the APOA2 protein variants when the human APOA2 protein variants comprising the amino acid sequence represented by SEQ ID NO: 1, 2 or 3 are used as the antigens, or antibody fragments of these antibodies. Alternatively, their chemically modified derivatives may be used. In this context, the "chemically modified derivatives" also include, for example, the anti-APOA2 protein terminus antibodies or the antibody fragments thereof functionally modified as required for acquiring or maintaining the specific binding activity for the particular APOA2 protein variant, or the anti-APOA2 protein terminus antibodies or the antibody fragments modified with labels necessary for detection.

Examples of the functional modification include glycosylation, deglycosylation, and PEGylation. Examples of the modification with labels include labeling with fluorescent dyes (FITC, rhodamine, Texas Red, Cy3, and Cy5), fluorescent proteins (e.g., PE, APC, GFP, and EGFP), enzymes (e.g., horseradish peroxidase, alkaline phosphatase, and glucose oxidase), biotin, avidin, or streptavidin.

The antibodies for use in the assay of the APOA2 protein variants may be any of polyclonal and monoclonal antibodies. Monoclonal antibodies are preferred for achieving specific detection. For example, an anti-APOA2 protein terminus polyclonal antibody specifically binding to the APOA2 protein terminus can be prepared by the aforementioned method.

The two APOA2 protein variants can be assayed by an immunological method using the anti-APOA2 antibodies each binding only to the particular APOA2 protein variant. The immunological method may be any method using the anti-APOA2 antibodies and is preferably ELISA using the anti-APOA2 protein terminus antibodies as immobilized antibodies or labeled antibodies which are combined with another antibody binding to a region other than the APOA2 protein C terminus (anti-APOA2 protein non-terminus antibody). For example, the amount of the APOA2-ATQ protein can be measured by sandwich ELISA using the anti-APOA2-ATQ terminus antibody as a labeled antibody and using the anti-APOA2-ATQ non-terminus antibody as an immobilized antibody. The APOA2-AT protein can be measured by sandwich ELISA using the anti-APOA2-AT terminus antibody as an immobilized antibody and using the anti-APOA2-AT non-terminus antibody as a labeled antibody. The anti-APOA2 protein non-terminus antibody is commercially available from Abcam plc., Fitzgerald Industries International, or the like, and such a commercially available product may be used.

2-2. Step of determining affection

The "step of determining affection" is the step of determining (or evaluating) *in vitro* whether to have pancreatic cancer or benign pancreatic tumor on the basis of the amounts of the proteins measured in the step of assaying the markers for pancreatic tumor detection. The amounts of the assayed markers for pancreatic tumor detection, i.e., the APOA2 protein variants (amounts of the APOA2-ATQ protein and the APOA2-AT protein), in the body fluid sample of a test subject are measured for pancreatic tumor detection to determine whether or not to have a pancreatic tumor or to evaluate the possibility of having a pancreatic tumor. This step comprises 3 steps (first to third steps). Hereinafter, each step will be described in detail.

In the first step, the amount of APOA2-ATQ protein in the body fluid sample of a test subject is measured using an anti-APOA2-ATQ terminus antibody specifically binding to a C-terminal region of the APOA2-ATQ protein comprising the amino acid sequence represented by SEQ ID NO: 1, and an anti-APOA2-ATQ non-terminus antibody binding to an amino acid sequence other than the C-terminal region.

Then, in the second step, the amount of APOA2-AT protein is measured using an anti-APOA2-AT terminus antibody specifically binding to a C-terminal region of the APOA2-AT protein comprising the amino acid sequence represented by SEQ ID NO: 2, and an anti-APOA2-AT non-terminus antibody binding to an amino acid sequence other than the C-terminal region. In this context, desirably, the C-terminal regions of the APOA2-ATQ protein and the APOA2-AT protein each consist of a sequence comprising 6 or more consecutive amino acids including the C terminus. The amounts of the APOA2 protein variants can be measured by, for example, ELISA, though the measurement method according to the present invention is not limited thereto. The anti-APOA2-ATQ non-terminus antibody used together with the anti-APOA2-ATQ terminus antibody in the first step may be identical as an anti-APOA2 protein non-terminus antibody to the anti-APOA2-AT non-terminus antibody used together with the anti-APOA2-AT terminus antibody in the second step. In short, the anti-APOA2-AT non-terminus antibody can also be used in the first step, while the anti-APOA2-ATQ non-terminus antibody can also be used in the second step.

In the third step, the measurement value of the amount of APOA2-ATQ protein obtained in the first step and the measurement value of the amount of APOA2-AT protein obtained in the second step are input to a preset discriminant to determine a discriminant value of the test subject. The test subject is determined to have a pancreatic tumor when this discriminant value is statistically significantly different as compared with the discriminant value of a normal subject. In this context, the discriminant used can be set by a method mentioned later.

Alternatively, even without determining the discriminant value, the human test subject may be conveniently determined to have a pancreatic tumor when the amount of either of the APOA2-ATQ protein or the APOA2-AT protein in the sample collected from the human test subject is significantly different from the amount thereof in a specimen collected from a normal person, specifically, significantly lower than the amount.

The method for detecting a pancreatic tumor according to the present invention can further comprise a fourth step as to the test subject determined to have a pancreatic tumor in the third step. In the fourth step, the amount of a known pancreatic cancer marker in a body fluid sample of this test subject can be measured to discriminately determine the test subject to have either of pancreatic cancer or benign pancreatic tumor. In this method, a maker known to be capable of detecting pancreatic cancer but incapable of detecting benign pancreatic tumor is used as the known pancreatic cancer marker. Specifically, a Sialyl-Lewis A antigen "CA19-9" (carbohydrate antigen 19-9) or a mucin-like glycoprotein "DU-PAN-2" (pancreatic cancer-associated antigen-2) can be used (LAB DATA: test selection and interpretation 2013-2014, supervised by Fumimaro Takaku, Igaku Shoin Ltd., p. 636-638). The reference value for pancreatic cancer discrimination is 37 (U/mL) for CA19-9 and 150 (U/mL) for DU-PAN-2. The amount of CA19-9 or DU-PAN-2 can be measured by, for example, ELISA, though the measurement method according to the present invention is not limited thereto.

The determination of pancreatic cancer or benign pancreatic tumor in the fourth step can be specifically carried out as follows: first, the amount of CA19-9 or DU-PAN-2 in the body fluid sample of the human test subject is measured. Next, the test subject can be determined to have pancreatic cancer when the measurement value of the amount of CA19-9 or DU-PAN-2 exceeds the corresponding reference value for pancreatic cancer discrimination. Also, the test subject can be determined to have benign pancreatic tumor when the measurement value is equal to or lower than this reference value. This is based on the fact that the method of the present invention for measuring the amounts of the APOA2 protein variants achieves detection of benign pancreatic tumor, which has previously been unattainable.

The method for detecting a pancreatic tumor according to the present invention can also be used in combination with an additional APOA2 protein variant such as the APOA2-A protein, or the total amount of the APOA2 proteins. Such an embodiment is also included in the scope of the present invention.

The "normal subject" refers to an individual at least having no pancreatic tumor, preferably a healthy individual. The normal subject is further required to be the same organism species as the test subject. When the test subject for the detection is, for example, a human (human test subject), the normal subject must also be a human (in the present specification, referred to as a "normal person" below). The physical conditions of the normal subject are preferably the same as or similar to those of the test subject. The physical conditions correspond to, for example, race, sex, age, height, and body weight for humans.

The concentrations of the markers for pancreatic tumor detection in the body fluid of the normal subject are preferably measured in the same way as the method for measuring the concentrations of the markers for pancreatic tumor detection in the body fluid of the test subject described in the step of assaying the markers for pancreatic tumor detection. The concentrations of the markers for pancreatic tumor detection in the body fluid of the normal subject may be measured every time the concentrations of the markers for pancreatic tumor detection in the body fluid of the test subject are measured. Alternatively, the concentrations of the markers for pancreatic tumor detection measured in advance can also be used. Particularly, the concentrations of the markers for pancreatic tumor detection are measured in advance under various physical conditions of normal subjects. The values are input to a computer and databased. This approach is convenient because the concentrations of the markers for pancreatic tumor detection of a normal subject having the optimum physical conditions for comparison with the test subject can be used at once by merely inputting the physical conditions of the test subject into the computer.

In the present specification, specific examples of the term "statistically significantly", for example, when the obtained value has a small critical value (significance level) include $p < 0.05$, $p < 0.01$, or $p < 0.001$. In this context, the term "p" or "p value" represents the probability of a statistical hypothesis being true by chance in the hypothesized distribution of statistics in a statistical test. Thus, smaller "p" or "p value" means that the hypothesis is closer to trueness. The phrase "statistically significantly different" means that there is a significant difference in the statistical processing of distinctive amounts of the markers for pancreatic tumor detection obtained from the test subject and the normal subject or distinctive discriminant values obtained by inputting the amounts to the discriminant. The test subject is evaluated as having a pancreatic tumor when being statistically significantly different as compared with the normal subject. A test method known in the art capable of determining the presence or absence of significance can be appropriately used as the test method for the statistical processing without particular limitations. For example, a Student's t test or multiple comparison test method can be used.

In the present specification, the "discriminant" is a final product of multivariate analysis and is characterized by one or more value sets. The discriminant value is eventually calculated according to this discriminant. In the present specification, the "multivariate analysis" is a mathematical approach that is used for constructing the discriminant by use of the measurement values of the markers for pancreatic tumor detection. In the present specification, the "value set" refers to a combination of values as to the features of the markers for pancreatic tumor detection, or a range of these values. This value set and the properties of the values in the set depend on the type of the features present in the markers for pancreatic tumor detection and the multivariate analysis that is used for constructing the discriminant defining the value set.

In the present specification, the "discriminant value" is a value that can be used as an index for making the prediction that the subject of interest would have a pancreatic tumor. As a specific example, the subject of interest can be predicted to have a pancreatic tumor by the discriminant value. As another example, the subject of interest can be predicted to have no pancreatic tumor by the discriminant value.

The discriminant can be constructed by multivariate analysis using data analysis algorithm. Examples of the data analysis algorithm that can be used in the construction of the discriminant include generalized linear models including logistic regression analysis, neural networks, support vector machines (SVM), discriminant analysis, nonparametric approaches, PLS (partial least squares), decision tress, principal component analysis, generalized additive models, fuzzy logic, SOM (self-organizing maps), and genetic algorithm. Among them, logistic regression analysis, a neural network, SVM, or discriminant analysis can be preferably used, though the data analysis algorithm according to the present invention is not limited thereto. The details of these statistical methods are found in the following references: Ruczinski, I. et al., 2003, Journal of Computational and Graphical Statistics, Vol. 12, p. 475-511; Friedman, J., Journal of the American Statistical Association, 1989, Vol. 84, p. 165-175; Hastie, T. et al., 2001, The Elements of Statistical Learning, Springer Series in Statistics; Breiman, L., 1984, Classification and regression trees, Chapman and Hall: Breiman, L., 2001, Machine Learning, Vol. 45, p. 5-32; Pepe, M., 2003, The Statistical Evaluation of Medical Tests for Classification and Prediction, Oxford Statistical Science Series; and Duda, R. et al., 2000, Pattern Classification, Wiley Interscience, the 2nd edition.

In the present invention, the analysis using the discriminant is conducted by the following steps: first, an event to be discriminated is set as an objective variable. The "objective variable" is an event to be discriminated according to the discriminant. In the present invention, the objective variable refers to whether or not a human test subject has a pancreatic tumor. In the case where the event to be discriminated by, for example, logistic regression analysis is whether or not a human test subject has a pancreatic tumor, the objective variable can be set to "1" when the human test subject is a pancreatic tumor patient, and to "0" when the human test subject is a normal person. Next, explanatory variables for predicting the objective variable are set. The "explanatory variables" are variables that are used for predicting the objective variable according to the discriminant. In the case of, for example, logistic regression analysis, the measurement values of the markers for pancreatic tumor detection, i.e., the APOA2-ATQ protein and the APOA2-AT protein, can be set as the explanatory variables. Next, a discriminant involving the explanatory variables in combination is constructed by use of any data analysis algorithm mentioned above, and a discriminant value is calculated. On the basis of the obtained discriminant value, the event to be discriminated is predicted. In the case of, for example, logistic regression analysis, the human test subject can be predicted to be a pancreatic tumor patient (i.e., "1") or a normal person (i.e., "0") from the discriminant value. Finally, the results of predicting the event are compared with the value of the objective variable to evaluate the discrimination performance of the discriminant. In this context, the "discrimination performance" refers to an index indicating to what degree the prediction of the event to be discriminated can be accurate. The discrimination outcomes (sensitivity and specificity) or AUC values of case data can be used as the discrimination performance. The discriminant value obtained from the discriminant is preferably used as a reference to determine whether or not to have a pancreatic tumor or to evaluate the possibility of having a pancreatic tumor.

In the present specification, the "AUC (area under the curve) value" means an area under a receiver operating characteristic curve (ROC curve) and serves as an index for measuring the accuracy of a prediction, determination, detection, or diagnosis method for classifying patients into a positive group and a negative group. In this curve, a value (false-positive rate) determined by subtracting the probability of producing positive results for positive patients (sensitivity) and the probability of producing negative results for negative patients (specificity) from 1 is plotted as to results produced by the method to be evaluated.

In the present specification, the "sensitivity" means a value of (the number of true positives) / (the number of true positives + the number of false-negatives). Higher sensitivity permits early detection of pancreatic cancer or detection of benign pancreatic tumor and leads to the complete resection of a lesion or reduction in the rate of recurrence.

In the present specification, the "specificity" means a value of (the number of true negatives) / (the number of true negatives + the number of false-positives). Higher specificity prevents needless additional tests ascribable to the misclassification of normal subjects into early pancreatic cancer patients or benign pancreatic tumor patients and leads to the alleviation of burdens on patients or reduction in medical cost.

Hereinafter, the method for analyzing whether or not a human test subject has a pancreatic tumor according to the discriminant based on logistic regression analysis using the measurement values of the APOA2 protein variants will be specifically described.

2-2-1. Discrimination method using logistic regression analysis

A method for obtaining a discriminant using logistic regression analysis can be used as the analysis method for determining whether or not to have a pancreatic tumor or evaluating the possibility of having a pancreatic tumor.

First, all human test subjects are divided according to clinical information into 2 groups: pancreatic tumor patients and normal persons. The objective variable is set to "1" for the pancreatic tumor patients and to "0" for the normal persons. Next, the discriminant is established from the measurement values of the two APOA2 protein variants obtained from biological samples having the clinical information. The discriminant can be preset as a logistic regression expression comprising, as a variable (explanatory variable), the measurement value of the APOA2-ATQ protein, and/or the measurement value of the APOA2-AT protein, and/or the product of the measurement value of the APOA2-AT protein and the measurement value of the APOA2-ATQ protein. The validity of the logistic regression expression as the discriminant can be evaluated by using an index such as AIC values (Akaike's information criterion) or Schwarz's BIC values belonging to the category of a maximum likelihood method.

An expression comprising the measurement value of the APOA2-ATQ protein, the measurement value of the APOA2-AT protein, and the product of the measurement value of the APOA2-AT protein and the measurement value of the APOA2-ATQ protein as explanatory variables, as in mathematical expression 1, mathematical expression 2, and mathematical expression 3, can be used as the logistic regression expression.

$$\text{Mathematical expression 1: } a \times (\text{APOA2-ATQ}) + b \times (\text{APOA2-AT}) + d$$

$$\text{Mathematical expression 2: } a \times (\text{APOA2-ATQ}) + b \times (\text{APOA2-AT}) + c \times (\text{APOA2-ATQ}) \times (\text{APOA2-AT}) + d$$

$$\text{Mathematical expression 3: } c \times (\text{APOA2-ATQ}) \times (\text{APOA2-AT}) + d$$

(In the mathematical expressions 1 to 3, a, b, c, and d each represent any real number except for zero; (APOA2-ATQ) represents the measurement value of the APOA2-ATQ protein; and (APOA2-AT) represents the measurement value of the APOA2-AT protein.)

In the case of obtaining the discriminant in the form of a logistic regression expression, the APOA2-ATQ protein and APOA2-AT protein measurement values obtained from a human test subject and a normal person are input to the logistic regression expression, and the resulting discriminant values can be compared to determine the human test subject to have a pancreatic tumor. For example, the human test subject can be determined to have a pancreatic tumor when the statistically significantly different discriminant value of the human test subject is 2/3 or lower, more preferably 1/2 or lower, further preferably 1/4 or lower, of the discriminant value of the normal person.

Examples

Hereinafter, the present invention will be described further specifically with reference to Examples below. However, the present invention is not intended to be limited by these Examples.

(Example 1) Preparation of monoclonal antibody specifically recognizing C-terminal region of APOA2-ATQ protein (anti-APOA2-ATQ terminus monoclonal antibody)

(a) Preparation of cell producing antibody recognizing C-terminal region of APOA2-ATQ protein

A peptide consisting of the amino acid sequence represented by SEQ ID NO: 29, which is the sequence of a C-terminal region of the APOA2-ATQ protein, is poorly soluble in water and low antigenic. Therefore, hydrophilicity was imparted thereto by the addition of 3 arginine residues to the N-terminal side, and a cysteine residue was further added to the N terminus to synthesize a peptide. Subsequently, OVA protein was bound to the cysteine residue of the peptide using Maleimide-Activated Ovalbumin (manufactured by Pierce Biotechnology, Inc.). The resulting peptide was intraperitoneally administered as an immunogen at a dose of 100 µg of the immunogen per mouse to mice (BALB/c) at 2-week intervals. For the first to fourth immunization shots, the immunogen solution was further mixed with Sigma Adjuvant System (manufactured by Sigma-Aldrich Corp.) and administered. After the fourth shot, the antibody titer in the mouse serum was measured by ELISA.

The immunogen was adjusted to 0.3 µg/mL with a PBS solution, then added at 100 µL/well to Immunoplate Maxisorp (manufactured by Nalge Nunc International), and immobilized overnight. On the next day, the solution was discarded, and blocking buffer A solution (0.5% BSA, 0.05% Tween 20, and PBS) was added thereto at 400 µL/well. The plate was left standing at room temperature for 1 hour. The solution in each well was discarded. Then, the well was washed by the addition of 400 µL of PBS-T (0.05% Tween 20 and PBS). The mouse serum diluted with blocking buffer A solution was added thereto at 100 µL/well and reacted at room temperature for 1 hour. The solution in each well was discarded. Then, the well was washed with PBS-T. Then, Polyclonal Rabbit Anti-Mouse Immunoglobulins/HRP (manufactured by Dako, An Agilent Company) diluted 5000-fold with blocking buffer A solution was added thereto at 100 µL/well and reacted at room temperature for 1 hour. After washing with PBS-T, a TMB solution (manufactured by Pierce Biotechnology, Inc.) was added thereto at 50 µL/well for enzymatic reaction. Then, the reaction was terminated by the addition of a 0.5 N sulfuric acid solution at 50 µL/well. The absorbance was measured at 450 nm. As a result, a sufficient rise in antibody titer was found. Therefore, the immunogen solution was administered to the mice for final immunization.

Three days after the final immunization date, antibody-producing cells were obtained from the spleen of each mouse.

(b) Recovery of antibody-producing cell from mouse and cell fusion

The antibody-producing cells obtained from each mouse in the step (a) and SP2/0 (mouse myeloma) cells were mixed at a ratio of 1:10 in an RPMI1640 medium and subjected to fusion reaction in the presence of 80% polyethylene glycol. Subsequently, the fusion cells were cultured for approximately 1 week in a HAT medium to select hybridomas.

(c) Selection of hybridoma producing antibody recognizing C-terminal region of APOA2-ATQ protein

Next, from the hybridomas selected in a HAT medium, antibodies specifically recognizing the C-terminal region of the APOA2-ATQ protein were selected by using, as an index, difference in binding activity against the recombinant human-derived APOA2-ATQ protein and APOA2-AT protein. As a result of screening by ELISA in the same way as in the step (a), two hybridomas, clone 7F2 and clone 6G2, were obtained. As a result of sequence analysis on genes encoding these monoclonal antibodies, the CDR sequences of 7F2 were shown to be the amino acid sequences represented by SEQ ID NOs: 4 to 9, and the CDR sequences of 6G2 were shown to be the amino acid sequences represented by SEQ ID NOs: 10 to 15.

(Example 2) Detection of APOA2-ATQ protein by ELISA using anti-APOA2-ATQ terminus monoclonal antibody

The anti-APOA2-ATQ terminus monoclonal antibody 7F2 or 6G2 obtained in Example 1 was used to detect the APOA2-ATQ protein by ELISA. The recombinant human-derived APOA2-ATQ protein, APOA2-AT protein, or APOA2-A protein was adjusted to 1 $\mu$g/mL with a PBS solution, then added at 100 $\mu$L/well to Immunoplate Maxisorp, and immobilized overnight. On the next day, the solution was discarded, and blocking buffer A solution was added thereto at 400 $\mu$L/well. The plate was left standing at room temperature for 1 hour. The solution in each well was discarded. Then, the well was washed by the addition of 400 $\mu$L of PBS-T. The antibody 7F2 or 6G2 diluted to 0.2 $\mu$g/mL with a diluent (1% NP40, 50 mM tris-HCl, 150 mM NaCl, 1 mM EDTA, and 1% BSA, pH 8.0) was added thereto at 100 $\mu$L/well and reacted at room temperature for 2 hours. The solution in each well was discarded. Then, after washing with PBS-T, Polyclonal Rabbit Anti-Mouse Immunoglobulins/HRP diluted 5000-fold with a diluent was added thereto at 100 $\mu$L/well and reacted at room temperature for 1 hour. After washing with PBS-T, a TMB solution was added thereto at 100 $\mu$L/well for enzymatic reaction. Then, the reaction was terminated by the addition of a 0.5 N sulfuric acid solution at 100 $\mu$L/well. The absorbance was measured at 450 nm.
The results are shown in Figure 1. Both of the anti-APOA2-ATQ terminus monoclonal antibody clone 7F2 and clone 6G2 obtained were confirmed to specifically recognize the APOA2-ATQ protein.

(Example 3) Evaluation of binding specificity of anti-APOA2-ATQ terminus monoclonal antibody and anti-APOA2-ATQ terminus polyclonal antibody against APOA2-ATQ protein

A total of 3 antibodies, i.e., the anti-APOA2-ATQ terminus monoclonal antibody clone 7F2 and clone 6G2 obtained in Example 1 and an anti-APOA2-ATQ terminus polyclonal antibody obtained by the method described in the paragraph "1-4. Preparation of anti-APOA2 polyclonal antibody" (the details of the method are described in "Experimental Protocol for Anti-Peptide Antibodies", the 2nd edition, Gakken Medical Shujunsha Co., Ltd.), were evaluated for their specificity for the antigen. In this experiment, POD-labeled forms of the clone 7F2, the clone 6G2, and the anti-APOA2-ATQ terminus polyclonal antibody were used. The POD labeling of the antibodies was carried out using PEROXIDASE LABELING KIT-SH (manufactured by Dojindo Laboratories).
The recombinant human-derived APOA2-ATQ protein or APOA2-AT protein was adjusted to 1 $\mu$g/mL with a PBS solution, then added at 100 $\mu$L/well to Immunoplate Maxisorp, and immobilized for 2 hours. After washing with PBS-T, blocking buffer B solution (1% skimmed milk, 0.05% Tween 20, and PBS) was added thereto at 400 $\mu$L/well. The plate was left standing at room temperature for 1 hour. The solution in each well was discarded. Subsequently, the POD-labeled form of the monoclonal antibody 7F2 or 6G2 or the anti-APOA2-ATQ terminus polyclonal antibody diluted to 0.5 $\mu$g/mL with a diluent was added thereto at 100 $\mu$L/well and reacted at room temperature for 2 hours. After further washing with PBS-T, a TMB solution was added thereto at 100 $\mu$L/well for color development. Finally, the reaction was terminated by the addition of a 0.5 N sulfuric acid solution at 100 $\mu$L/well. The absorbance was measured at 450 nm.
Figure 2(A) shows the measurement values of each antibody reacted with a protein-unimmobilized well (blank), the APOA2-AT protein-immobilized well, and the APOA2-ATQ protein-immobilized well. Figure 2(B) is a graph depicting a value obtained as the binding specificity of each anti-APOA2-ATQ terminus antibody by multiplying its binding activity value for the APOA2-ATQ protein by its binding activity value for the APOA2-AT protein, wherein the binding activity value for the APOA2-AT protein was calculated by subtracting the measurement value of the antibody reacted with the APOA2-AT protein-immobilized well from the blank, and the binding activity value for the APOA2-ATQ protein was calculated by subtracting the measurement value of the antibody reacted with the APOA2-ATQ protein-immobilized well from the blank. Both of the anti-APOA2-ATQ terminus monoclonal antibodies 7F2 and 6G2 were confirmed to have stronger binding specificity for the APOA2-ATQ protein than that of the anti-APOA2-ATQ terminus polyclonal antibody.

(Example 4) Detection of APOA2-AT protein by ELISA using anti-APOA2-AT terminus antibody

The APOA2-AT protein was detected by ELISA using an anti-APOA2-AT terminus polyclonal antibody specifically recognizing the C-terminal region of the APOA2-AT protein.

(a) Preparation of anti-APOA2-AT terminus polyclonal antibody

The anti-APOA2-AT terminus polyclonal antibody was obtained by use of the method described in the paragraph "1-4. Preparation of anti-APOA2 polyclonal antibody" (the details of the method are described in "Experimental Protocol for Anti-Peptide Antibodies", the 2nd edition, Gakken Medical Shujunsha Co., Ltd.). The immunogen used was prepared by adding a cysteine residue to the N terminus of a peptide consisting of the APOA2-AT protein C-terminal region shown in SEQ ID NO: 28 and further binding a carrier protein KLH thereto. This immunogen was administered to rabbits at 1-week intervals. After the fourth shot, the antibody titer in the rabbit serum was measured by ELISA in the same way as in Example 1. As a result, a sufficient rise in antibody titer was found. Therefore, antiserum was recovered 1 week after the final immunization date.

A formyl-Cellulofine carrier was bound to the peptide and used as an affinity column to purify the antiserum. Specifically, the antiserum after purification was passed through the affinity column (formyl-Cellulofine carrier bound with a C-terminally amidated form of the peptide) so that immunoglobulins exhibiting binding activity against a region other than the C-terminal region of the peptide were removed by adsorption. Finally, this non-adsorbed fraction was obtained as the anti-APOA2-AT terminus polyclonal antibody.

(b) Detection of APOA2-AT protein using ELISA

The recombinant human-derived APOA2-AT protein, APOA2-ATQ protein, or APOA2-A protein was adjusted to 1 $\mu$g/mL with a PBS solution, then added at 100 $\mu$L/well to Immunoplate Maxisorp, and immobilized overnight. On the next day, the solution was discarded, and blocking buffer A solution was added thereto at 400 $\mu$L/well. The plate was left standing at room temperature for 1 hour. The solution in each well was discarded. Then, the well was washed by the addition of 400 $\mu$L of PBS-T. The anti-APOA2-AT terminus polyclonal antibody diluted to 0.2 $\mu$g/mL with a diluent was added thereto at 100 $\mu$L/well and reacted at room temperature for 2 hours. The solution in each well was discarded. Then, after washing with PBS-T, Anti-Rabbit IgG, HRP-Linked F(ab')$_2$ Fragment Donkey (manufactured by GE Healthcare Japan Corp.) diluted 10000-fold with a diluent was added thereto at 100 $\mu$L/well and reacted at room temperature for 1 hour. After washing with PBS-T, a TMB solution was added thereto at 100 $\mu$L/well for enzymatic reaction. Then, the reaction was terminated by the addition of a 0.5 N sulfuric acid solution at 100 $\mu$L/well. The absorbance was measured at 450 nm.

The results are shown in Figure 3. The antibody was confirmed to specifically recognize only the APOA2-AT protein among the APOA2 protein variants.

(Example 5) Detection of APOA2 protein by ELISA using anti-APOA2 protein non-terminus antibody

Antibodies recognizing an amino acid sequence other than the C-terminal region of the APOA2 protein were prepared by use of the method described in Example 1.

Hybridomas were screened by using, as an index, binding activity against the APOA2 protein non-terminus region to select antibodies. As a result of this screening, hybridomas producing two anti-APOA2 protein non-terminus monoclonal antibodies, clone MAB1 and clone MAB2, were obtained. As a result of sequence analysis on genes encoding these monoclonal antibodies, the CDR sequences of MAB1 were shown to be the amino acid sequences represented by SEQ ID NOs: 16 to 21, and the CDR sequences of MAB2 were shown to be the amino acid sequences represented by SEQ ID NOs: 22 to 27.

The anti-APOA2 protein non-terminus monoclonal antibody MAB1 or MAB2 was used to detect the APOA2 protein variants by ELISA. The recombinant human-derived APOA2-AT protein, APOA2-ATQ protein, or APOA2-A protein was adjusted to 1 $\mu$g/mL with a PBS solution, then added at 100 $\mu$L/well to Immunoplate Maxisorp, and immobilized overnight. On the next day, the solution was discarded, and blocking buffer A solution was added thereto at 400 $\mu$L/well. The plate was left standing at room temperature for 1 hour. The solution in each well was discarded. Then, the well was washed by the addition of 400 $\mu$L of PBS-T. Each antibody diluted to 0.5 $\mu$g/mL with blocking buffer A was added thereto

at 100 μL/well and reacted at room temperature for 1 hour. The solution in each well was discarded. Then, after washing with PBS-T, Anti-Mouse IgG, HRP-Linked F(ab')$_2$ Fragment Donkey (manufactured by GE Healthcare Japan Corp.) diluted 5000-fold with blocking buffer A was added thereto at 100 μL/well and reacted at room temperature for 1 hour. After washing with PBS-T, a TMB solution was added thereto at 100 μL/well for enzymatic reaction. Then, the reaction was terminated by the addition of a 0.5 N sulfuric acid solution at 100 μL/well. The absorbance was measured at 450 nm.

Figures 4(A) and 4(B) show the results of assaying the APOA2 protein variants by ELISA using the antibodies MAB1 and MAB2, respectively. Both of the monoclonal antibodies MAB1 and MAB2 had the same levels of binding activity against the APOA2 protein variants and were therefore confirmed to be anti-APOA2 protein non-terminus antibodies recognizing an amino acid sequence other than the C-terminal region.

(Comparative Example 1) Detection of APOA2 protein dimer (APOA2-ATQ/AT) in blood using mass spectrometry

The ion strength of a peptide peak having a mass of 17252 (m/z) was measured by use of SELDI-QqTOF-MS (surface-enhanced laser desorption/ionization high-resolution performance hybrid quadrupole time of flight mass spectrometry) according to an approach similar to Experiment 1 described in JP Patent No. 5200246 from plasma collected from 40 subjects each of pancreatic cancer patients and normal persons by their informed consent in National Cancer Center Hospital.

Figure 5 shows the results of discriminating the pancreatic cancer patients from the normal persons. This approach exhibited AUC value = 0.894 and was thus confirmed to have high pancreatic cancer discrimination accuracy.

(Comparative Example 2) Detection of APOA2 protein dimer (APOA2-ATQ/AT) in blood using sandwich ELISA

Plasma obtained in the same way as in Comparative Example 1 was used as a sample. The detection of the APOA2 protein dimer (APOA2-ATQ/AT) was attempted by sandwich ELISA using the anti-APOA2-ATQ terminus monoclonal antibody 7F2 specifically recognizing the C-terminal region of the APOA2-ATQ protein and a POD-labeled form of the anti-APOA2-AT terminus polyclonal antibody specifically recognizing the C-terminal region of the APOA2-AT protein.

The POD labeling of the anti-APOA2-ATQ terminus monoclonal antibody was carried out using PEROXIDASE LABELING KIT-SH, and the details followed the attached protocol. The anti-APOA2-AT terminus polyclonal antibody was adjusted to 5 μg/mL with a PBS solution, then added at 100 μL/well to Immunoplate Maxisorp, and immobilized overnight. On the next day, the solution was discarded, and the well was washed by the addition of 400 μL of PBS-T. Blocking buffer C solution (1% BSA, 0.05% Tween 20, and PBS) was added thereto at 400 μL/well, and the plate was left standing at room temperature for 1 hour. The solution was discarded to prepare an antibody-immobilized plate. Next, the plasma diluted 16-fold with a diluent was added thereto at 100 μL/well and reacted at room temperature for 1 hour. The solution in each well was discarded. Then, the well was washed with PBS-T. The POD-labeled form of the anti-APOA2-ATQ terminus monoclonal antibody diluted to 0.4 μg/mL with a diluent was added thereto at 100 μL/well and reacted at room temperature for 1 hour. After washing with PBS-T, a TMB solution was added thereto at 100 μL/well for enzymatic reaction. Then, the reaction was terminated by the addition of a 0.5 N sulfuric acid solution at 100 μL/well. The absorbance was measured at 450 nm.

Figure 6 shows the results of discriminating the pancreatic cancer patients from the normal persons. This approach exhibited AUC value = 0.529 and thus, did not offer pancreatic cancer discrimination accuracy equivalent to the mass spectrometry of Comparative Example 1. The results of this experiment indicate the possibility that because two C-terminal regions of the APOA2-ATQ/AT protein dimer are positioned in proximity to each other, the anti-APOA2-AT terminus polyclonal antibody and the anti-APOA2-ATQ terminus monoclonal antibody cannot bind thereto at the same time due to steric hindrance.

(Comparative Example 3) Detection of APOA2-ATQ protein in blood using sandwich ELISA

Plasma obtained in the same way as in Comparative Example 1 was used as a sample. The APOA2-ATQ protein was assayed by sandwich ELISA using a POD-labeled form of the anti-APOA2-ATQ terminus monoclonal antibody 7F2 and an anti-APOA2 protein non-terminus polyclonal antibody (Fitzgerald Industries International) recognizing a site other than the C-terminal region of the APOA2 protein.

The POD labeling of the antibody 7F2 was carried out in the same way as in Comparative Example 2. The anti-APOA2 protein non-terminus polyclonal antibody was adjusted to 2 μg/mL with a PBS solution, then added at 100 μL/well to Immunoplate Maxisorp, and immobilized overnight. On the next day, the solution was discarded, and the well was washed by the addition of 400 μL of PBS-T. Blocking buffer C solution was added thereto at 400 μL/well, and the plate was left standing at room temperature for 1 hour. Then, the solution was discarded to prepare an antibody-immobilized plate. Next, the plasma diluted 10000-fold with a diluent was added thereto at 100 μL/well and reacted at room temperature for 1 hour. The antigen solution in each well was discarded. Then, the well was washed with PBS-T. The POD-labeled form of the antibody 7F2 diluted to 0.2 μg/mL with a diluent was added thereto at 100 μL/well and reacted at room temperature for 1 hour. After washing with PBS-T, a TMB solution was added thereto at 100 μL/well for enzymatic reaction. Then, the reaction was terminated by the addition of a 0.5 N sulfuric acid solution at 100 μL/well. The absorbance was measured at 450 nm.

Figure 7 shows the results of discriminating the pancreatic cancer patients from the normal persons. This approach exhibited AUC value = 0.515 and thus, did not produce excellent pancreatic cancer discrimination accuracy.

(Comparative Example 4) Detection of APOA2-AT protein in blood using sandwich ELISA

Plasma obtained in the same way as in Comparative Example 1 was used as a sample. The APOA2-AT protein was assayed by sandwich ELISA using the anti-APOA2-AT terminus polyclonal antibody and a POD-labeled form of the anti-APOA2 protein non-terminus polyclonal antibody. The POD labeling of the anti-APOA2 protein non-terminus polyclonal antibody and the sandwich ELISA were carried out in the same way as in Comparative Example 3. The dilution ratio of the plasma was set to 6000-fold.

Figure 8 shows the results of discriminating the pancreatic cancer patients from the normal persons. This approach exhibited AUC value = 0.814 and was thus confirmed to have high pancreatic cancer discrimination accuracy, which was however inferior in discrimination performance to the mass spectrometry of Comparative Example 1.

(Example 6) Pancreatic cancer discrimination by combination of measurement values of two APOA2 proteins (APOA2-ATQ protein and APOA2-AT protein) in blood

Figure 9 shows results of discriminating pancreatic cancer patients from normal persons by plotting the product of the measurement values of the APOA2-ATQ protein and the APOA2-AT protein obtained in Comparative Example 3 and Comparative Example 4, respectively. This approach exhibited AUC value = 0.906 and was thus confirmed to exhibit high pancreatic cancer discrimination accuracy, which was superior even to the mass spectrometry of Comparative Example 1.

(Example 7) Pancreatic cancer discrimination using sandwich ELISA

Plasma collected from 244 pancreatic cancer patients and 109 normal persons by their informed consent in National Cancer Center Hospital was used as a sample. Two APOA2 protein variants (APOA2-ATQ protein and APOA2-AT protein) in blood were assayed according to the approach of Example 6. Antigen solutions of the recombinant human-derived proteins APOA2-ATQ protein and APOA2-AT protein were used as preparations to calculate the concentrations of the two proteins in the plasma.

Figure 10 shows a scatter diagram on which the two proteins in the plasma of each subject were plotted. Use of the two proteins was shown to be able to discriminate the pancreatic cancer patients from the normal persons with high accuracy.

(Example 8) Pancreatic cancer discrimination by processing of measurement value with data analysis algorithm

A discriminant that offers high pancreatic cancer discrimination accuracy can be obtained by the statistical analysis processing of the measurement values obtained in Example 7. Statistical processing given below was carried out and compared with the same pancreatic cancer discrimination method using mass spectrometry as in the Comparative Example 1.

(a) Pancreatic cancer discrimination using logistic regression analysis

The objective variable was defined as "1" for the pancreatic cancer patients and as "0" for the normal persons. The concentrations of the two APOA2 protein variants (APOA2-ATQ protein and APOA2-AT protein) in blood obtained in (1) were used as explanatory variables in logistic regression analysis to calculate discriminants. Table 1 shows the calculation results about AUC values and discrimination outcomes (sensitivity and specificity) of case data in the obtained discriminants. In this table, the good discrimination performance of early pancreatic cancer (stage I) was obtained in the discriminant constructed by using the product of the two protein concentrations (APOA2-ATQ protein and APOA2-AT protein) as an explanatory variable, as in Example 6. The measurement values of the APOA2-ATQ protein and the APOA2-AT protein in each specimen were input to this discriminant, and the resulting discriminant value was used to discriminate pancreatic cancer patients (stages I and II) from normal persons. The obtained ROC curve is shown in Figure 11(A). Figure 11(B) is an ROC curve obtained from the discrimination of the pancreatic cancer patients from the normal persons using the amount of the APOA2-ATQ/AT protein dimer measured by the mass spectrometry. Table 2 shows results of comparing discrimination performance for each stage of pancreatic cancer. In Table 2, the stage of pancreatic cancer follows the UICC stage classification: stage I refers to IA and IB according to the UICC stage classification; and stage II refers to IIA and IIB according to the UICC stage classification. In Table 2, "ELISA" depicts the analysis results obtained from the discriminant by using, as an explanatory variable, the product of the amounts of the two proteins (APOA2-ATQ protein and APOA2-AT protein) obtained by ELISA. "Mass spectrometry" depicts the analysis results obtained using the amount of the APOA2 protein dimer (APOA2-ATQ/AT) obtained by mass spectrometry. This approach was confirmed to be capable of detecting early pancreatic cancer with very high sensitivity as compared with the mass spectrometry.

[Table 1]

| Explanatory variable | AUC | Detection sensitivity of early cancer (stage I) (%) | Detection sensitivity of cancers including all stages (%) | Specificity (%) |
|---|---|---|---|---|
| AT | 0.823 | 57 | 75 | 71 |
| AT×ATQ | 0.904 | 86 | 80 | 85 |
| AT, ATQ | 0.921 | 71 | 87 | 82 |
| AT, AT×ATQ | 0.926 | 71 | 87 | 84 |
| ATQ, AT×ATQ | 0.920 | 71 | 86 | 83 |

AT and ATQ represent the measurement values of the APOA2-AT protein and the APOA2-ATQ protein, respectively, in ELISA assay. AT×ATQ represents the product of these measurement values.

[Table 2]

| | The number of cases: head-count | Sensitivity: % (head-count) | |
|---|---|---|---|
| | | ELISA | Mass spectrometry |
| Pancreatic cancer patient | 244 | 80(194) | 78(190) |
| Stage | | | |
| I | 7 | 86(6) | 71(5) |
| II | 33 | 85(28) | 70(23) |
| III | 69 | 77(53) | 78(54) |
| IV | 135 | 79(107) | 80(198) |

| | The number of cases: head-count | Specificity: % (head-count) | |
|---|---|---|---|
| | | ELISA | Mass spectrometry |
| | 109 | 85(93) | 81(88) |

(Example 9) Discrimination of benign pancreatic tumor by processing of measurement value with data analysis algorithm

APOA2 was used in the discrimination of various benign pancreatic tumor patients from normal persons and compared with CA19-9 as to discrimination performance.

The same discriminant (the product of the amounts of the APOA2-ATQ protein and the APOA2-AT protein) as that used for discriminating the pancreatic cancer patients from the normal persons in Example 8 was used in the discrimination using APOA2. The discrimination performance obtained using APOA2 was calculated in the same way as in the calculation of discrimination outcomes (sensitivity and specificity) in Table 1.

The amount of CA19-9 in the body fluid samples of the human test subjects was measured by an immunological method. Typically, the discrimination reference value is 37 (U/mL) for discriminating pancreatic cancer patients from normal persons using CA19-9. By the discrimination, the human test subjects are determined to be normal persons when the amount of CA19-9 is equal to or lower than the reference value, and determined to be pancreatic cancer patients when the amount of CA19-9 exceeds the reference value (LAB DATA: test selection and interpretation 2013-2014, supervised by Fumimaro Takaku, Igaku Shoin Ltd., p. 636-637). This Example was intended to test whether benign pancreatic tumor patients could be discriminated from normal persons using CA19-9 on the basis of the reference value. For this purpose, each test subject was determined to be a benign pancreatic tumor patient by the discrimination when the amount of CA19-9 exceeded the reference value.

Figure 12(A) shows an ROC curve obtained from the discrimination of the benign pancreatic tumor patients from the normal persons using APOA2. Figure 12(B) shows an ROC curve obtained from the discrimination of the benign pancreatic tumor patients from the normal persons using CA19-9. Table 3 shows results of comparing discrimination performance of each benign pancreatic tumor. The discrimination method of the present invention using APOA2 was confirmed to be capable of detecting benign pancreatic tumor with very high sensitivity. By contrast, use of CA19-9 offered sensitivity almost equal to zero and thus turned out to have the difficulty in detecting benign pancreatic tumor.

[Table 3]

| | The number of cases: head-count | Sensitivity: % APOA2 | (head-count) CA19-9 |
|---|---|---|---|
| Intraductal papillary mucinous adenoma of pancreas | 17 | 76(13) | 6(1) |
| Mucinous cystic adenoma of pancreas | 4 | 100(4) | 0(0) |
| Neuroendocrine tumor of pancreas | 7 | 100(7) | 14(1) |
| Serous cystadenoma of pancreas | 3 | 100(3) | 0(0) |
| Atypical hyperplasia and carcinoma *in situ* | 2 | 100(2) | 0(0) |
| Total | 33 | 88(29) | 6(2) |

(Example 10) Discrimination of pancreatic cancer and benign pancreatic tumor by combination of APOA2 and CA19-9

Pancreatic cancer and benign pancreatic tumor were discriminated by the combination of APOA2 and CA19-9. First, benign pancreatic tumor, early pancreatic cancer (stages I and II), and pancreatic cancer (all stages) were detected by the methods described in Examples 8 and 9 using APOA2. The number of affected persons in which any of the diseases was detected was calculated as the number of APOA2 positives. Next, the APOA2-positive patients were subjected to discrimination using CA19-9 by the method described in Example 9. The number of persons in which the amount of CA19-9 exceeded the reference value was calculated as the number of CA19-9 positives. The ratio of the number of CA19-9 positives to the number of APOA2 positives was further calculated. These results are shown in Table 4.

This approach was confirmed to have the high probability that the APOA2-positive and CA19-9-positive patients can be determined to have pancreatic cancer while the APOA2-positive and CA19-9-negative patients can be determined to have benign pancreatic tumor. These results demonstrated that according to the method of the present invention, test subjects can be discriminately determined to have pancreatic cancer or benign pancreatic tumor by the combination of APOA2 and CA19-9.

[Table 4]

| | The number of APOA2 positives: head-count | The number of CA19-9 positives*: head-count | The number of CA19-9 positives* / The number of APOA2 positives |
|---|---|---|---|
| Benign pancreatic tumor | 29 | 2 | 7% |
| Pancreatic cancer (stages I and II) | 34 | 22 | 65% |
| Pancreatic cancer (all stages) | 194 | 151 | 78% |

* represents the number of CA19-9-positive affected persons accounting for APOA2-positive affected persons

(Example 11) Discrimination of pancreatic cancer and benign pancreatic tumor by combination of APOA2 and DU-PAN-2

Pancreatic cancer and benign pancreatic tumor were discriminated by the combination of APOA2 and DU-PAN-2. The discrimination was carried out in the same way as the method described in Example 10 except that DU-PAN-2 was used instead of CA19-9. The amount of DU-PAN-2 in the body fluid samples of the test subjects was measured by an immunological method. Typically, the discrimination reference value is 150 (U/mL) for discriminating pancreatic cancer patients from normal persons using DU-PAN-2. By the discrimination, the test subjects are determined to be normal persons when the amount of DU-PAN-2 is equal to or lower than the reference value, and determined to be pancreatic cancer patients when the amount of DU-PAN-2 exceeds the reference value (LAB DATA: test selection and interpretation 2013-2014, supervised by Fumimaro Takaku, Igaku Shoin Ltd., p. 637-638). In this Example, the discrimination was carried out using this reference value of DU-PAN-2. The results are shown in Table 5.

[Table 5]

|  | The number of APOA2 positives: head-count | The number of DU-PAN-2 positives*: head-count | The number of DU-PAN-2 positives* |
| --- | --- | --- | --- |
|  |  |  | The number of APOA2 positives |
| Benign pancreatic tumor | 29 | 4 | 14% |
| Pancreatic cancer (stages I and II) | 34 | 14 | 41% |
| Pancreatic cancer (all stages) | 194 | 128 | 66% |
| * represents the number of DU-PAN-2-positive affected persons accounting for APOA2-positive affected persons | | | |

This approach was confirmed to have the high probability that the APOA2-positive and DU-PAN-2-positive patients can be determined to have pancreatic cancer while the APOA2-positive and DU-PAN-2-negative patients can be determined to have benign pancreatic tumor. These results demonstrated that according to the method of the present invention, test subjects can be discriminately determined to have pancreatic cancer or benign pancreatic tumor by the combination of APOA2 and DU-PAN-2.

The results of Examples 7 to 11 described above demonstrated that the method of the present invention is useful in the highly sensitive detection of pancreatic cancer including early pancreatic cancer (stages I and II) or benign pancreatic tumor, which has previously been considered to be difficult, by analysis using a discriminant based on the measurement values of the APOA2 variants. These results also demonstrated that the method of the present invention is useful in achieving the highly accurate discrimination between pancreatic cancer and benign pancreatic tumor, which has previously been unattainable, by analyzing the detection results in combination with the pancreatic cancer marker (CA19-9 or DU-PAN-2).

Industrial Applicability

According to the present invention, a pancreatic tumor can be detected with high throughput by a simple and noninvasive method. As a result, the early detection of the pancreatic tumor is realized.

**Claims**

1.  A method for detecting pancreatic cancer or benign pancreatic tumor by measuring the amounts of apolipoprotein A2 (APOA2) protein variants in a body fluid sample of a test subject, the detection method comprising:

    (A) a first step of measuring the amount of APOA2-ATQ protein in the sample using an anti-APOA2-ATQ terminus antibody specifically binding to a C-terminal region of the APOA2-ATQ protein comprising the amino acid sequence represented by SEQ ID NO: 1, and an anti-APOA2-ATQ non-terminus antibody binding to the amino acid sequence other than the C-terminal region;
    (B) a second step of measuring the amount of APOA2-AT protein in the sample using an anti-APOA2-AT terminus antibody specifically binding to a C-terminal region of the APOA2-AT protein comprising the amino acid sequence represented by SEQ ID NO: 2 and an anti-APOA2-AT non-terminus antibody binding to the amino acid sequence other than the C-terminal region; and
    (C) a third step of inputting, to a preset discriminant, the measurement value of the amount of APOA2-ATQ protein obtained in the first step and the measurement value of the amount of APOA2-AT protein obtained in the second step, and determining the test subject to have pancreatic cancer or benign pancreatic tumor when the resulting discriminant value of the test subject is statistically significantly different as compared with the discriminant value of a normal subject,

    wherein the APOA2-ATQ protein is the full length APOA2 protein and the APOA2-AT protein is the APOA2 protein lacking the C-terminal glutamine residue.

2.  The detection method according to claim 1, wherein the C-terminal regions of the APOA2-ATQ protein and the APOA2-AT protein each consist of a sequence comprising 6 or more consecutive amino acids including the C terminus.

3.  The detection method according to claim 1 or 2, wherein the discriminant is any one selected from the group consisting of a logistic regression expression, an expression prepared by analysis with a support vector machine, an expression prepared by the analysis of a neural network, and an expression prepared by discriminant analysis.

4.  The detection method according to claim 3, wherein the logistic regression expression comprises, as a variable, the measurement value of the APOA2-ATQ protein, the measurement value of the APOA2-AT protein, and/or the product of the measurement value of the APOA2-ATQ protein and the measurement value of the APOA2-AT protein.

**5.** The method according to claim 4, wherein the discriminant value of the test subject obtained from the logistic regression expression is 2/3 or lower of the discriminant value of a normal subject.

**6.** The detection method according to any one of claims 1 to 5, further comprising a fourth step of measuring the amount of a pancreatic cancer marker CA19-9 or DU-PAN-2 in a body fluid sample of the test subject determined to have pancreatic cancer or benign pancreatic tumor in the third step, and determining the test subject to have pancreatic cancer when the measurement value exceeds a predetermined reference value and determining the test subject to have benign pancreatic tumor when the measurement value is equal to or lower than the reference value.

**7.** The detection method according to any one of claims 1 to 6, wherein the body fluid sample is blood, plasma, or serum.

**8.** The detection method according to any one of claims 1 to 7, wherein the pancreatic cancer is early pancreatic cancer.

**Patentansprüche**

**1.** Verfahren zum Nachweis von Bauchspeicheldrüsenkrebs oder gutartigem Tumor der Bauchspeicheldrüse durch Messen der Mengen an Apolipoprotein A2 (APOA2)-Proteinvarianten in einer Körperflüssigkeitsprobe einer Testperson, wobei das Nachweisverfahren umfasst:

(A) einen ersten Schritt des Messens der Menge des APOA2-ATQ-Proteins in der Probe unter Verwendung eines Anti-APOA2-ATQ-Terminus-Antikörpers, der spezifisch an eine C-terminale Region des APOA2-ATQ-Proteins bindet, umfassend die durch SEQ ID NO: 1 dargestellte Aminosäuresequenz, und eines Anti-APOA2-ATQ-Nichtterminus-Antikörpers, der an eine andere Aminosäuresequenz als die C-terminale Region bindet;
(B) einen zweiten Schritt des Messens der Menge des APOA2-AT-Proteins in der Probe unter Verwendung eines Anti-APOA2-AT-Terminus-Antikörpers, der spezifisch an eine C-terminale Region des APOA2-AT-Proteins bindet, umfassend die durch SEQ ID NO: 2 dargestellte Aminosäuresequenz, und eines Anti-APOA2-AT-Nichtterminus-Antikörper, der an eine andere Aminosäuresequenz als die C-terminalen Region bindet; und
(C) einen dritten Schritt der Eingabe des Messwertes der im ersten Schritt erhaltenen Menge an APOA2-ATQ-Protein und des Messwertes der im zweiten Schritt erhaltenen Menge an APOA2-AT-Protein in eine voreingestellte Diskriminante, und Bestimmens, dass die Testperson Bauchspeicheldrüsenkrebs oder einen gutartigen Tumor der Bauchspeicheldrüse hat, wenn der resultierende Diskriminanzwert der Testperson statistisch signifikant anders ist als der Diskriminanzwert einer normalen Testperson, worin das APOA2-ATQ-Protein das APOA2-Protein in voller Länge ist und das APOA2-AT-Protein das APOA2-Protein ohne den C-terminalen Glutaminrest ist.

**2.** Nachweisverfahren nach Anspruch 1, worin die C-terminalen Bereiche des APOA2-ATQ-Proteins und des APOA2-AT-Proteins jeweils aus einer Sequenz bestehen, die 6 oder mehr aufeinanderfolgende Aminosäuren einschließlich des C-Terminus umfasst.

**3.** Nachweisverfahren nach Anspruch 1 oder 2, wobei die Diskriminante irgendeine ist, die ausgewählt ist aus der Gruppe bestehend aus einem logistischen Regressionsausdruck, einem Ausdruck, der durch Analyse mit einer Trägervektormaschine erzeugt wurde, einem Ausdruck, der durch die Analyse eines neuronalen Netzwerks erzeugt wurde, und einem Ausdruck, der durch Diskriminanzanalyse erzeugt wurde.

**4.** Nachweisverfahren nach Anspruch 3, worin der logistische Regressionsausdruck als Variable den Messwert des APOA2-ATQ-Proteins, den Messwert des APOA2-AT-Proteins und/oder das Produkt aus dem Messwert des APOA2-ATQ-Proteins und dem Messwert des APOA2-AT-Proteins umfasst.

**5.** Verfahren nach Anspruch 4, worin der Diskriminanzwert der Testperson, der aus dem logistischen Regressionsausdruck erhalten wird, 2/3 oder niedriger als der Diskriminanzwert einer normalen Testperson ist.

**6.** Nachweisverfahren nach einem der Ansprüche 1 bis 5, ferner umfassend einen vierten Schritt des Messens der Menge eines Bauchspeicheldrüsenkrebsmarkers CA19-9 oder DU-PAN-2 in einer Körperflüssigkeitsprobe der Testperson, von dem im dritten Schritt bestimmt wurde, Bauchspeicheldrüsenkrebs oder einen gutartigen Tumor der Bauchspeicheldrüse zu haben, und des Bestimmens, dass die Testperson Bauchspeicheldrüsenkrebs hat, wenn der Messwert einen vorbestimmten Referenzwert überschreitet, und des Bestimmens, dass die Testperson einen gutartigen Tumor der Bauchspeicheldrüse hat, wenn der Messwert gleich oder niedriger als der Referenzwert ist.

**7.** Nachweisverfahren nach einem der Ansprüche 1 bis 6, worin die Körperflüssigkeitsprobe Blut, Plasma oder Serum ist.

**8.** Nachweismethode nach einem der Ansprüche 1 bis 7, worin der Bauchspeicheldrüsenkrebs ein früher Bauchspeicheldrüsenkrebs ist.

**Revendications**

**1.** Procédé de détection d'un cancer pancréatique ou d'une tumeur pancréatique bénigne par la mesure des quantités de variants protéique de l'apolipoprotéine A2 (APOA2) dans un échantillon de fluide corporel d'un sujet testé, le procédé de détection comprenant :

(A) une première étape de mesure de la quantité de protéine APOA2-ATQ dans l'échantillon en utilisant un anticorps terminal anti-APOA2-ATQ se liant spécifiquement à une région C-terminale de la protéine APOA2-ATQ comprenant la séquence d'acides aminés représentée par SEQ ID NO : 1, et un anticorps non terminal anti-APOA2-ATQ se liant à la séquence d'acides aminés autre que la région C-terminale ;
(B) une deuxième étape de mesure de la quantité de protéine APOA2-AT dans l'échantillon en utilisant un anticorps terminal anti-APOA2-AT se liant spécifiquement à une région C-terminale de la protéine APOA2-AT comprenant la séquence d'acides aminés représentée par SEQ ID NO : 2 et un anticorps non terminal anti-APOA2-AT se liant à la séquence d'acides aminés autre que la région C-terminale ; et
(C) une troisième étape d'entrée, dans un discriminant prédéfini, de la valeur de mesure de la quantité de protéine APOA2-ATQ obtenue dans la première étape et de la valeur de mesure de la quantité de protéine APOA2-AT obtenue dans la deuxième étape, et de détermination que le sujet testé présente un cancer pancréatique ou une tumeur pancréatique bénigne quand la valeur de discriminant résultante du sujet testé est statistiquement significativement différente par rapport à la valeur de discriminant d'un sujet normal,

dans lequel la protéine APOA2-ATQ est la protéine APOA2 de pleine longueur et la protéine APOA2-AT est la protéine APOA2 dépourvue du résidu glutamine C-terminal.

**2.** Procédé de détection selon la revendication 1, dans lequel les régions C-terminales de la protéine APOA2-ATQ et de la protéine APOA2-AT consistent chacune en une séquence comprenant 6 acides aminés ou plus consécutifs incluant l'extrémité C-terminale.

**3.** Procédé de détection selon la revendication 1 ou 2, dans lequel le discriminant est l'un quelconque sélectionné dans le groupe constitué d'une expression de régression logistique, d'une expression préparée par une analyse avec une machine à vecteurs de support, une expression préparée par l'analyse d'un réseau de neurones, et une expression préparée par une analyse discriminante.

**4.** Procédé de détection selon la revendication 3, dans lequel l'expression de régression logistique comprend, comme variable, la valeur de mesure de la protéine APOA2-ATQ, la valeur de mesure de la protéine APOA2-AT, et/ou le produit de la valeur de mesure de la protéine APOA2-ATQ et de la valeur de mesure de la protéine APOA2-AT.

**5.** Procédé selon la revendication 4, dans lequel la valeur de discriminant du sujet testé obtenue à partir de l'expression de régression logistique représente 2/3 ou moins de la valeur de discriminant d'un sujet normal.

**6.** Procédé de détection selon l'une quelconque des revendications 1 à 5, comprenant en outre une quatrième étape de mesure de la quantité d'un marqueur de cancer pancréatique CA19-9 ou DU-PAN-2 dans un échantillon de fluide corporel du sujet testé déterminé pour avoir un cancer pancréatique ou une tumeur pancréatique bénigne dans la troisième étape, et de détermination que le sujet testé présente un cancer pancréatique quand la valeur de mesure dépasse une valeur de référence prédéterminée et de détermination que le sujet testé présente une tumeur pancréatique bénigne quand la valeur de mesure est inférieure ou égale à la valeur de référence.

**7.** Procédé de détection selon l'une quelconque des revendications 1 à 6, dans lequel l'échantillon de fluide corporel est du sang, du plasma, ou du sérum.

**8.** Procédé de détection selon l'une quelconque des revendications 1 à 7, dans lequel le cancer pancréatique est un cancer pancréatique précoce.

# Fig. 1

(A)

(B)

# Fig. 2

(A)

(B)

Fig. 3

# Fig. 4

(A)

(B)

# Fig. 5

Fig. 6

# Fig. 7

Fig. 8

# Fig. 9

# Fig. 10

# Fig. 11

(A) ELISA (AUC=0.904)
(B) Mass spectrometry (AUC=0.810)

## Fig. 12

(A) APOA2  (AUC=0.944)
(B) CA19-9  (AUC=0.498)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2001289861 A
- JP 2002323499 A
- JP 2009034071 A
- WO 2006098087 A
- JP 2010175452 A
- US 5714350 A
- US 6350861 B
- WO 9713844 A
- WO 9002809 A
- JP 5200246 B
- JP 2014166188 A
- JP 2013206682 A

### Non-patent literature cited in the description

- Clinical Practice Guidelines for Pancreatic Cancer based on evidence-based medicine. Japan Pancreas Society, 2009
- **PANKHURST G. et al.** *J. Lipid Res.,* 2003, vol. 44, 349-355
- **BLANCO-VACA F. et al.** *J. Lipid Res.,* 2001, vol. 42, 1727-1739
- **ROCCO AG. et al.** *Biophys J.,* 2006, vol. 91, 3043-3049
- **HONDA K. et al.** *PLoS One,* 2012, vol. 7, e46908
- General Rules for the Study of Pancreatic Cancer. Japan Pancreas Society, 2013
- TNM Classification of Malignant Tumours. TNM Committee of the Japan National Committee for UICC, 2012
- **E.A. KABAT et al.** Sequences of proteins of immunological interest. NIH, 1991, vol. 1
- Fundamental Immunology. 1993
- Pierce catalog and Handbook. Pierce Chemical co, 1994-1995
- **HOLLIGER et al.** *Proc. Natl. Acad. Sci USA,* 1993, vol. 90, 6444-6448
- **MARVIN et al.** *Acta Pharmacol. Sin.,* 2005, vol. 26, 649-658
- **ALAFSEN et al.** *Prot. Engr. Des. Sel.,* 2004, vol. 17, 21-27
- **MCCAFFERTY et al.** *Nature,* 1990, vol. 348, 552-554
- **KUBY, J.** Immunology. W.H. Freeman & Co, 1998
- *Journal of immunology,* 1998, vol. 160, 3393-3402
- **GREENE ; SAMBROOK.** Molecular Cloning: A Laboratory Manual. Cold Spring Harbor Laboratory Press, 2012
- *PNAS,* 1989, vol. 86, 6077
- *PNAS,* 1987, vol. 84, 7413
- *Virology,* 1973, vol. 52, 456-467
- *Nature,* 1977, vol. 266, 550-552
- **BRINKMANN et al.** *J. Immunol Methods,* 1995, vol. 182, 41-50
- Experimental Medicine, Suppl.: Handbook of Gene Engineering. Yodosha Co., Ltd, 1991, 297-299
- *European Journal of Cancer Prevention,* 1996, vol. 5, 512-519
- *Cancer Research,* 1990, vol. 50, 1495-1502
- Zoku-Seikagaku Jikken Koza 1, Idenshi Kenkyuho II (Experiments in Biochemistry, second series, Methods in Gene research in English). Japanese Biochemical Society, 289-305
- Experimental Protocol for Anti-Peptide Antibodies. Gakken Medical Shujunsha Co., Ltd
- **RUCZINSKI, I. et al.** *Journal of Computational and Graphical Statistics,* 2003, vol. 12, 475-511
- **FRIEDMAN, J.** *Journal of the American Statistical Association,* 1989, vol. 84, 165-175
- **HASTIE, T. et al.** The Elements of Statistical Learning. Springer Series in Statistics, 2001
- Classification and regression trees. **BREIMAN, L.** Machine Learning. Chapman and Hall, 1984, vol. 45, 5-32
- **PEPE, M.** The Statistical Evaluation of Medical Tests for Classification and Prediction. *Oxford Statistical Science Series,* 2003
- **DUDA, R. et al.** Pattern Classification. Wiley Interscience, 2000
- Experimental Protocol for Anti-Peptide Antibodies. Gakken Medical Shujunsha Co., Ltd,

SEQUENCE LISTING

<110> TORAY Industries, Inc. National Cancer Center

<120> Method of detecting pancreatic cancer, antibodies and detection kit for pancreatic cancer

<130> PH-5996-PCT

<150> JP 2014-166188
<151> 2014-08-18

<150> JP 2013-206682
<151> 2013-10-01

<160> 29

<170> PatentIn version 3.5

<210> 1
<211> 77
<212> PRT
<213> Homo sapiens

<400> 1

```
Gln Ala Lys Glu Pro Cys Val Glu Ser Leu Val Ser Gln Tyr Phe Gln
1               5                   10                  15

Thr Val Thr Asp Tyr Gly Lys Asp Leu Met Glu Lys Val Lys Ser Pro
            20                  25                  30

Glu Leu Gln Ala Glu Ala Lys Ser Tyr Phe Glu Lys Ser Lys Glu Gln
        35                  40                  45

Leu Thr Pro Leu Ile Lys Lys Ala Gly Thr Glu Leu Val Asn Phe Leu
    50                  55                  60

Ser Tyr Phe Val Glu Leu Gly Thr Gln Pro Ala Thr Gln
65                  70                  75
```

<210> 2
<211> 76
<212> **PRT**
<213> Homo sapiens

<400> 2

```
Gln Ala Lys Glu Pro Cys Val Glu Ser Leu Val Ser Gln Tyr Phe Gln
1               5                   10                  15

Thr Val Thr Asp Tyr Gly Lys Asp Leu Met Glu Lys Val Lys Ser Pro
            20                  25                  30

Glu Leu Gln Ala Glu Ala Lys Ser Tyr Phe Glu Lys Ser Lys Glu Gln

        35                  40                  45

Leu Thr Pro Leu Ile Lys Lys Ala Gly Thr Glu Leu Val Asn Phe Leu
    50                  55                  60

Ser Tyr Phe Val Glu Leu Gly Thr Gln Pro Ala Thr
65                  70                  75
```

<210> 3
<211> 75

<212> PRT
<213> Homo sapiens

<400> 3

```
Gln Ala Lys Glu Pro Cys Val Glu Ser Leu Val Ser Gln Tyr Phe Gln
1               5                   10                  15


Thr Val Thr Asp Tyr Gly Lys Asp Leu Met Glu Lys Val Lys Ser Pro
            20                  25                  30


Glu Leu Gln Ala Glu Ala Lys Ser Tyr Phe Glu Lys Ser Lys Glu Gln
            35                  40                  45


Leu Thr Pro Leu Ile Lys Lys Ala Gly Thr Glu Leu Val Asn Phe Leu
        50                  55                  60


Ser Tyr Phe Val Glu Leu Gly Thr Gln Pro Ala
65                  70                  75
```

<210> 4
<211> 10
<212> PRT
<213> Artificial Sequence

<220>
<223> antibody

<400> 4

```
Gly Tyr Thr Phe Thr Asn Tyr Trp Met His
1               5                   10
```

<210> 5
<211> 17
<212> PRT
<213> Artificial Sequence

<220>
<223> antibody

<400> 5

```
Asn Ile Tyr Pro Gly Ser Gly Asn Thr Asn Tyr Asn Glu Lys Phe Lys
    1           5                   10                      15


    Ser
```

<210> 6
<211> 10
<212> PRT
<213> Artificial Sequence

<220>
<223> antibody

<400> 6

Arg Tyr Gly Tyr Val Asp Trp Phe Ala Tyr
1               5               10

<210> 7
<211> 16
<212> PRT
<213> Artificial Sequence

<220>
<223> antibody

<400> 7

Arg Ser Ser Lys Ser Leu Leu Tyr Lys Asp Gly Lys Thr Tyr Leu Asn
1               5               10                  15

<210> 8
<211> 7
<212> PRT
<213> Artificial Sequence

<220>
<223> antibody

<400> 8

Leu Met Ser Thr Arg Ala Ser
1               5

<210> 9
<211> 9
<212> PRT
<213> Artificial Sequence

<220>
<223> antibody

<400> 9

Gln Gln Leu Val Glu Tyr Pro Leu Thr
1               5

<210> 10
<211> 5
<212> PRT
<213> Artificial Sequence

<220>
<223> antibody

<400> 10

Asn Tyr Gly Met Asn 1 5

<210> 11
<211> 17
<212> PRT
<213> Artificial Sequence

<220>
<223> antibody

<400> 11

```
Trp Lys Asn Thr Tyr Thr Gly Glu Ser Thr Tyr Ala Asp Asp Phe Lys
1               5                   10                  15

Gly
```

<210> 12
<211> 11
<212> PRT
<213> Artificial Sequence

<220>
<223> antibody

<400> 12

```
Arg Asp Gly Ser Lys Tyr Lys Ile Phe Asp Tyr
1               5                   10
```

<210> 13
<211> 12
<212> PRT
<213> Artificial Sequence

<220>
<223> antibody

<400> 13

```
Arg Ala Ser Ser Ser Leu Ser Ser Ser Tyr Leu His
1               5                   10
```

<210> 14
<211> 7
<212> PRT
<213> Artificial Sequence

<220>
<223> antibody

<400> 14

```
Ser Thr Ser Asn Leu Ala Ser
1               5
```

<210> 15

<211> 9
<212> PRT
<213> Artificial Sequence

<220>
<223> antibody

<400> 15

Gln Gln Phe Ser Val Phe Pro Leu Thr
1               5

<210> 16
<211> 10
<212> PRT
<213> Artificial Sequence

<220>
<223> antibody

<400> 16

Gly Tyr Thr Phe Thr Ser Tyr Trp Met His
1               5                   10

<210> 17
<211> 17
<212> PRT
<213> Artificial Sequence

<220>
<223> antibody

<400> 17

Phe Ile Asn Pro Ser Thr Gly Tyr Thr Glu Asn Asn Gln Arg Phe Asn
1               5                   10                  15

Asp

<210> 18
<211> 10
<212> PRT
<213> Artificial Sequence

<220>
<223> antibody

<400> 18

Arg Pro Tyr Asn Pro Tyr Ala Met Asp Tyr
1               5                   10

<210> 19
<211> 11
<212> PRT
<213> Artificial Sequence

<220>
<223> antibody

<400> 19

```
                        Arg Ala Ser Gln Asp Thr Ser Asn Tyr Leu Asn
                        1               5                   10
```

<210> 20
<211> 7
<212> PRT
<213> Artificial Sequence

<220>
<223> antibody

<400> 20

```
                        Tyr Thr Ser Arg Leu His Ser
                        1               5
```

<210> 21
<211> 9
<212> PRT
<213> Artificial Sequence

<220>
<223> antibody

<400> 21

```
                    Gln Gln Gly Asn Thr Leu Pro Tyr Thr
                    1               5
```

<210> 22
<211> 10
<212> PRT
<213> Artificial Sequence

<220>
<223> antibody

<400> 22

```
                    Gly Tyr Thr Phe Thr Ser Tyr Trp Met His
                    1               5                   10
```

<210> 23
<211> 17
<212> PRT
<213> Artificial Sequence

<220>
<223> antibody

<400> 23

```
Phe Ile Asn Pro Ser Thr Gly Tyr Thr Glu Asn Asn Gln Asn Phe Lys
1               5                   10                  15
```

```
Glu
```

<210> 24
<211> 10
<212> PRT
<213> Artificial Sequence

<220>
<223> antibody

<400> 24

```
Arg Thr Tyr Asn Pro Tyr Gly Met Asp Tyr
1               5                   10
```

<210> 25
<211> 11
<212> PRT
<213> Artificial Sequence

<220>
<223> antibody

<400> 25

```
Arg Ala Ser Gln Asp Ile Ser Asn Tyr Leu Asn
1               5                   10
```

<210> 26
<211> 7
<212> PRT
<213> Artificial Sequence

<220>
<223> antibody

<400> 26

```
Tyr Thr Ser Arg Leu Gln Ser
1               5
```

<210> 27
<211> 9
<212> PRT
<213> Artificial Sequence

<220>
<223> antibody

<400> 27

```
Gln Gln Gly Asn Thr Leu Pro Tyr Thr
1               5
```

```
<210> 28
<211> 16
<212> PRT
<213> Homo sapiens

<400> 28
```

```
        Val Asn Phe Leu Ser Tyr Phe Val Glu Leu Gly Thr Gln Pro Ala Thr
        1               5                   10                  15
```

```
<210> 29
<211> 17
<212> PRT
<213> Homo sapiens

<400> 29
```

```
        Val Asn Phe Leu Ser Tyr Phe Val Glu Leu Gly Thr Gln Pro Ala Thr
        1               5                   10                  15
```

```
        Gln
```